(19) Europäisches Patentamt
European Patent Office
Office européen des brevets

(11) EP 2 849 810 B1

(12) **FASCICULE DE BREVET EUROPEEN**

(45) Date de publication et mention de la délivrance du brevet:
**03.07.2019 Bulletin 2019/27**

(21) Numéro de dépôt: **12791802.7**

(22) Date de dépôt: **30.11.2012**

(51) Int Cl.:
***A61L 27/54*** *(2006.01)* ***A61L 27/58*** *(2006.01)*
***A61K 8/73*** *(2006.01)* ***A61L 27/08*** *(2006.01)*
***A61L 27/20*** *(2006.01)* ***A61L 31/04*** *(2006.01)*
***A61L 24/08*** *(2006.01)* ***A61Q 19/08*** *(2006.01)*

(86) Numéro de dépôt international:
**PCT/EP2012/074059**

(87) Numéro de publication internationale:
**WO 2013/079646 (06.06.2013 Gazette 2013/23)**

(54) **SOLUTION AQUEUSE HOMOGENE DE CHITOSANE INJECTABLE**

HOMOGENE WÄSSERIGE LÖSUNG VON INJIZIERBAREM CHITOSAN

HOMOGENEOUS AQUEOUS SOLUTION OF INJECTABLE CHITOSAN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.11.2011 FR 1160988**

(43) Date de publication de la demande:
**25.03.2015 Bulletin 2015/13**

(73) Titulaires:
- **BIOXIS Pharmaceuticals**
**69007 Lyon (FR)**
- **Université Jean-Monnet**
**42023 Saint Etienne (FR)**
- **Institut National des Sciences Appliquées de Lyon**
**69100 Villeurbanne (FR)**
- **Université Claude Bernard Lyon 1**
**69100 Villeurbanne (FR)**
- **Centre National de la Recherche Scientifique (C.N.R.S.)**
**75016 Paris (FR)**

(72) Inventeurs:
- **DUPASQUIER, Florence**
**71600 Paray Le Monial (FR)**
- **DAVID, Laurent**
**69004 Lyon (FR)**
- **DELAIR, Thierry**
**69700 Echalas (FR)**

(74) Mandataire: **Cabinet Plasseraud**
**66, rue de la Chaussée d'Antin**
**75440 Paris Cedex 09 (FR)**

(56) Documents cités:
**WO-A1-2009/150651 FR-A1- 2 897 775**

- **M. DASH, F. CHIELLINI, R.M. OTTENBRITE, E. CHIELLINI: "Chitosan - A versatile semi-synthetic polymer in biomedical applications", PROGRESS IN POLYMER SCIENCE, vol. 36, 22 février 2011 (2011-02-22), pages 981-1014, XP002678924,**
- **ESAM A. EL-HEFIAN, ABDUL H. YAHAYA: "Rheological study of chitosan and its blends: an overview.", MAEJO INT. J. SCI. TECHNOL., 2010, pages 210-220, XP002678925,**
- **PANOS INES ET AL: "New Drug Delivery Systems Based on Chitosan", CURRENT DRUG DISCOVERY TECHNOLOGIES, BENTHAM SCIENCE PUBLISHERS, NL, vol. 5, no. 4, 1 décembre 2008 (2008-12-01), pages 333-341, XP008151715, ISSN: 1570-1638**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 849 810 B1

Printed by Jouve, 75001 PARIS (FR)

## Description

La présente invention concerne le domaine technique des produits de comblement ou des biomatériaux, injectables chez l'homme ou l'animal. En particulier, la présente invention concerne une solution aqueuse homogène de chitosane injectable étant apte à former des particules cristallines de chitosane après injection. La présente invention concerne également des compositions contenant une telle solution aqueuse homogène de chitosane. L'invention a également pour objet de telles compositions pour leur utilisation comme composition dermatologique, cosmétique, ou encore comme dispositif médical, avantageusement comme implant biorésorbable.

Différents produits de comblement injectables notamment chez l'être humain sont déjà connus.

Le collagène a longtemps été le produit de choix comme produit de comblement pour le visage, en particulier pour le comblement des rides et des ridules ou encore pour réourler les lèvres. Cependant, depuis la mise sur le marché des acides hyaluroniques, ces derniers sont les plus utilisés. En effet, à la biodégradabilité du collagène jugée trop rapide, s'ajoutent les problèmes de sécurité liés à l'origine animale (bovine ou porcine) de celui-ci.

L'injection directe d'acide hyaluronique présente deux avantages : un effet de comblement mécanique immédiat et une absence de phénomènes inflammatoires, du fait de sa biocompatibilité. Toutefois, cette biocompatibilité va de pair avec une biodégradation rapide qui rend le produit insatisfaisant, même si la durée de vie du produit injecté a pu être prolongée grâce à l'utilisation d'acide hyaluronique réticulé.

Néanmoins, les produits les plus utilisés aujourd'hui en médecine et chirurgie esthétique sont des produits résorbables dont la durée de vie est généralement inférieure à 12 mois.

On trouve encore sur le marché des produits de comblement qu'on peut qualifier de « permanents », dans le sens où leur biorésorption peut nécessiter plusieurs années. Ces produits contiennent entre autres des polymères synthétiques ou biosynthétiques, tels que des dérivés acryliques, des polyacrylamides, qui induisent une encapsulation fibreuse importante à l'origine de la longévité du comblement. Toutefois, la persistance du produit injecté dans les tissus présente un risque de complications à long terme ou de phénomènes inflammatoires à retardement, par exemple la formation de granulomes inflammatoires, kystes... plusieurs mois, voire plusieurs années, après leur injection.

D'autres produits constituent aujourd'hui une alternative intéressante : il s'agit de PLA (acide polylactique), un polymère dont la biodégradation est plus lente que celle des autres polymères naturels, tels que le collagène ou l'acide hyaluronique. On estime en effet que le comblement persiste jusqu'à deux ans après l'injection. Ces produits sont commercialisés notamment sous le nom de New-Fill (ou Sculptra). Le principal défaut de cette technologie est que l'effet de comblement n'est visible qu'après un délai de huit semaines, ce qui n'apporte pas une complète satisfaction au patient.

Par ailleurs, la fibrose observée lors de l'utilisation de produits non dégradables est apparue de grand intérêt en terme d'effet esthétique à long terme, et c'est ainsi qu'ont été développés des produits de comblement qualifiés de « semi-permanents » qui, par leur composition hétérogène « particules-vecteur» ont un effet pro-fibrotique tout en demeurant biodégradables. On citera par exemple le produit Atlean qui propose une dispersion de particules de TCP (phosphate tricalcique) dans de l'acide hyaluronique et le produit Radiesse qui propose une dispersion de particules d'hydroxyapatite de calcium dans un gel de carboxyméthyle cellulose. Dans tous les cas, le gel vecteur assure l'effet esthétique de comblement immédiat, tandis que les particules génèrent petit à petit une fibrose qui garantit l'effet à long terme. L'intérêt de ces produits, outre ce double mécanisme d'action (mécanique et inducteur de tissu), est qu'ils sont finalement totalement résorbés.

De manière particulièrement avantageuse, le chitosane, de part sa structure chimique unique, se comporte vis-à-vis de l'organisme comme un « leurre » du milieu biologique (A. Montembault, K. Tahiri, C. Korwin-Zmijowska, X, Chevalier, M. Corvol, A.Domard, Biochimie, 88 (2006), 551-64): d'une part, il est suffisamment « reconnu » pour ne pas induire de réaction inflammatoire dangereuse, et d'autre part suffisamment « méconnu » pour ne pas être dégradé trop rapidement.

La molécule est en effet constituée d'une succession de fragments N-acétyl-D-Glucosamine et D-glucosamine, le premier étant un constituant de molécules de la matrice extracellulaire (on trouve ce résidu dans l'acide hyaluronique par exemple), et le second étant complètement absent de celle-ci, le chitosane est donc plus difficile à dégrader d'un point de vue biologique.

Le concept de l'utilisation d'un « leurre » du milieu biologique est tout à fait nouveau dans le domaine des injectables notamment en médecine esthétique, et aucun produit de comblement contenant du chitosane n'est commercialisé à ce jour.

Par ailleurs, le chitosane est connu dans la littérature pour stimuler certaines cellules de l'immunité, telles que les macrophages, qui produisent en sa présence une quantité accrue de facteurs de croissance. Ces facteurs de croissance sont des médiateurs biologiques qui favorisent la production de matrice extracellulaire et la prolifération des fibroblastes, cellules productrices des fibres de collagène. Ainsi, le chitosane favorise la synthèse de tissu fibreux, ce qui permet un comblement « biologique » à long terme et sans effets secondaires indésirables, notamment un comblement des défauts cutanés ou des cavités du corps humain ou du visage, telles que les rides.

La présente invention a ainsi pour objet une solution aqueuse homogène de chitosane injectable contenant

un chitosane ayant un degré d'acétylation inférieur à 20%, avantageusement inférieur à 10% et une masse moléculaire moyenne en masse comprise entre 100 000 et 1 000 000 g/mol, ladite solution contenant entre 0,1 et 3,5 %, avantageusement entre 1 et 2,5 %, en poids de chitosane, ladite solution présentant un pH inférieur à 6,2, avantageusement compris entre 5 et 6,2, ladite solution ne contenant pas de chitosane ayant un degré d'acétylation supérieur à 20%, ladite solution ne contenant pas un autre chitosane de plus faible masse moléculaire moyenne et ladite solution aqueuse étant apte à former des particules cristallines de chitosane après injection.

Le chitosane est un amino-polysaccaharide généralement obtenu par N-désacétylation de la chitine, polysaccharide aussi répandu dans la biomasse que la cellulose. La chitine est notamment présente dans les cuticules des arthropodes, l'endosquelette des céphalopodes, les parois cellulaires ou encore la matrice extracellulaire de champignons, levures ou algues.

Avantageusement selon la présente invention, le chitosane est un produit naturel qui provient d'une source animale, par exemple de crustacés du type crabes, crevettes ou calmars, ou d'une source végétale, telle que des champignons ou des algues.

Le chitosane et la chitine sont des copolymères linéaires respectivement de 2-acetamido-2-desoxy-D-glucane et de 2-amino-2-desoxy-D-glucane. On parle plus communément d'unités N-acétyl-D-glucosamine (GlcNAc) et D-Glucosamine (GlcN)), liées par des liaisons glycosidiques $\beta$-(1$\rightarrow$4). Chitine et chitosane se différencient par la fraction molaire (exprimée en %) des unités GlcNAc présentes dans le copolymère, appelée aussi degré d'acétylation (DA).

Les structures chimiques du chitosane et de la chitine sont représentées schématiquement ci-dessous en fonction du degré d'acétylation (DA) :

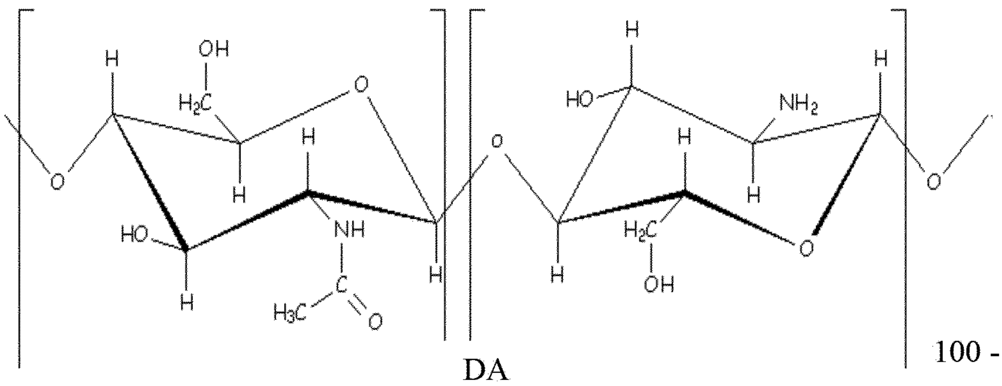

DA 100 - DA

N-acétyl-D-Glucosamine (GlcNAc) D-Glucosamine(GlcN)

Degré d'acétylation (DA) :

$$DA(\%) = \frac{nGlcNAc}{nGlcNAc + nGlcN} \times 100$$

avec nGlcNAc = nombre de motifs acétylés et nGlcN = nombre de motifs désacétylés.

Avantageusement selon la présente invention, le chitosane a un degré d'acétylation (DA) inférieur à 20%, encore plus avantageusement inférieur ou égal à 15%, par exemple inférieur à 10%. Typiquement, le chitosane selon l'invention a un degré d'acétylation (DA) compris entre 0,5 et 20 %, typiquement entre 1 et 15 %, par exemple entre 2 et 10%.

Typiquement, le chitosane a une masse moléculaire moyenne en masse (déterminée comme décrit dans «Physico-chemical studies of the gelation of chitosan in a hydroalcoholic médium» A. MONTEMBAULT, C. VITON, A. DOMARD Biomaterials, 26(8), 933-943, 2005) comprise entre 100 000 et 1 000 000 g/mol, avantageusement entre 250 000 et 1 000 000 g/mol, par exemple entre 250 000 et 500 000 g/mol, par exemple entre 250 000 et 400 000 g/mol.

De manière particulièrement avantageuse, le pH de la solution aqueuse selon la présente invention est inférieur à 6,2, et est typiquement compris entre 5 et 6,2. Dans le cadre de l'invention, le chitosane est soluble en solution aqueuse, telle que l'eau, dans un environnement acide dans les gammes de pH mentionnées précédemment, avantageusement par protonation des groupes amine du chitosane. Avantageusement, la solution aqueuse selon l'invention est stable.

Par solution « homogène » de chitosane, on entend au sens de la présente invention que tout le polymère chitosane est solubilisé, ne contenant pas de particules solides en suspension dans la phase liquide. La solution selon l'invention n'est ainsi pas gélifiée. La solution selon l'invention est ainsi typiquement transparente.

Selon une caractéristique particulière de la présente invention, la solution aqueuse homogène de chitosane contient entre 0,1 et 3,5 %, avantageusement entre 0,5 et 3,5 %, en particulier entre 1 et 2,5 %, en poids de chitosane, par rapport au poids total de la solution aqueuse.

De manière particulièrement avantageuse, la solution aqueuse selon l'invention est injectable dans le corps humain ou animal, typiquement par voie intradermique ou sous-cutanée. La solution peut être conditionnée dans une seringue telle qu'une seringue stérile.

Avantageusement, la solution aqueuse présente une viscosité appropriée pour assurer une bonne seringuabilité (écoulement satisfaisant à travers une aiguille dans une seringue) et une facilité d'injection.

Dans un mode de réalisation particulier, la solution aqueuse selon l'invention est stérilisée avant injection, par exemple par autoclave.

Après stérilisation, le chitosane a typiquement une masse moléculaire moyenne en masse comprise entre 120 000 et 400 000 g/mol, et avantageusement comprise entre 120 000 et 300 000 g/mol.

De manière particulièrement avantageuse, la solution aqueuse selon la présente invention avant injection ne contient pas de chitosane ayant un degré d'acétylation supérieur à 20%. Ainsi, le chitosane selon l'invention n'est pas mélangé avec un chitosane ayant un degré d'acétylation compris entre 30 et 60%, tel que cela est décrit dans les demandes de brevet WO 2008/072230 et WO 2009/150651.

Dans un mode de réalisation particulier selon la présente invention, la solution aqueuse contient plusieurs chitosanes, mais avec un seul degré d'acétylation (DA), ledit degré d'acétylation étant inférieur à 20%, avantageusement inférieur à 10%.

Dans un autre mode de réalisation particulier de la présente invention, la solution aqueuse contient à titre de polymère un seul chitosane ayant un degré d'acétylation tel que défini précédemment, ayant avantageusement une masse moléculaire moyenne telle que définie précédemment, avantageusement à une teneur comprise entre 0,1 et 3,5 %, avantageusement entre 0,5 et 3,5 %, en particulier entre 1 et 2,5 %, en poids de chitosane, par rapport au poids total de la solution aqueuse.

Dans un mode de réalisation particulier, la solution aqueuse selon l'invention peut être partiellement réticulée par interactions ioniques induites par exemple par l'ajout de sulfate, citrate, anions métalliques ou encore molécules anioniques, en particulier par la formation de complexes polyélectrolytes avec des polysaccharides présentant un groupe carboxylique $COO^-$ (alginates, pectine, xanthane, acide hyaluronique), avec des polysaccharides possédant un groupe sulfate, ou avec l'acide polylactique (PLA), ou encore par interaction avec des protéines (le collagène), des acides nucléiques (l'ADN, l'ARN, les Si ARN, les mARN...) ou des polysaccharides oxydés.

Dans un autre mode de réalisation particulier, la solution aqueuse selon l'invention est partiellement réticulée à l'aide d'agents réticulants covalents (ex : génipine), à l'exclusion d'agents connus pour leur toxicité, tels que des agents du groupe des époxy ou esters bi ou poly fonctionnels, de la divinyl sulfone, des carbodiimides, et des dialdéhydes.

Avantageusement, l'agent réticulant, qu'il soit de type ionique ou covalent, est introduit de manière à ce que le taux de réticulation soit suffisamment faible pour ne pas altérer l'aptitude de la solution aqueuse à former des particules cristallines de chitosane après injection.

Dans un mode de réalisation particulier, la solution aqueuse selon l'invention est composée de l'association d'une solution aqueuse de chitosane non réticulée avec une solution aqueuse de chitosane réticulée.

Selon une caractéristique particulière, la solution aqueuse selon l'invention est susceptible d'être préparée par les étapes suivantes :

- dissolution du chitosane dans l'eau par ajout d'acide organique tel qu'un acide faible, ledit acide faible étant avantageusement choisi dans le groupe constitué par l'acide acétique, l'acide glycolique, l'acide lactique, l'acide glutamique, et leurs mélanges,
- et éventuellement réajustement du pH pour obtenir une solution aqueuse présentant un pH compris entre 5 et 6,2, typiquement entre 5 et 5,5.

Avant dissolution, le chitosane est typiquement sous forme de poudre. Après dissolution, le chitosane est sous forme protonée. Il s'agit d'un polyélectrolyte cationique dont le contre-ion est issu de l'acide utilisé pour la dissolution. Par exemple, si l'acide acétique est ajouté à l'eau pour dissoudre le chitosane, on retrouve le chitosane sous forme d'acétate de chitosane, c'est-à-dire une forme protonée $NH_3^+$ des fonctions amines en interaction électrostatique avec les ions acétates.

Le contrôle du pH des solutions est très important pour éviter la nécrose acide des tissus après injection, et également pour protéger les solutions de l'hydrolyse et la dégradation du chitosane si l'on met en oeuvre une stérilisation (par exemple par autoclave à 121°C pendant 15 minutes).

Le pH est réajusté si nécessaire avec un composé tel que le bicarbonate de sodium ou un tampon PBS (« Phosphate Buffer Saline » - solution saline de tampon phosphate), typiquement dans des quantités réduites. La valeur du pH est avantageusement contrôlée grâce à un pH-mètre pendant la remontée de pH pour rester à un pH inférieur à

6,2 et éviter la gélification de la solution.

Dans un mode de réalisation particulier selon l'invention, le chitosane est dissous dans l'eau à l'aide d'un acide fort du type acide chlorhydrique. Dans ce cas, le pH est réajusté avec un composé de type bicarbonate de sodium ou d'ammonium ou PBS par exemple, et/ou une base de type NaOH ou KOH par exemple (toujours en contrôlant le pH de manière à ce qu'il reste inférieur à 6,2).

Dans un mode de réalisation particulier selon l'invention, lors de l'étape de dissolution, l'acide est ajouté en une quantité nécessaire à la dissolution du chitosane. On peut utiliser ainsi un excès d'acide pour certains chitosanes, par exemple les chitosane difficiles à solubiliser avec la quantité strictement nécessaire d'acide, puis on reprécipite le chitosane, à l'aide d'ammoniaque par exemple. Après une série de lavages destinés à éliminer l'excédent d'ammoniaque et les sels, on peut alors lyophiliser le chitosane pour récupérer la matière sèche. Celle-ci sera alors plus facile à solubiliser.

Dans un autre mode de réalisation particulier selon l'invention, lors de l'étape de dissolution, l'acide est ajouté en une quantité strictement nécessaire à la dissolution du chitosane, telle que la quantité stoechiométrique strictement nécessaire à la protonation des sites $NH_2$.

Typiquement, le nombre de sites à protoner est calculé de la manière suivante :

$$Mmonomère = 203 \times DA + 161 \times (1 - DA)$$

$$N_{NH2} = \frac{m \times (1-DA) \times (1-\%eau)}{Mmonomère}$$

avec m = masse de matière première introduite, %eau = teneur en eau de la matière première, DA = degré d'acétylation.

Après injection notamment dans le derme ou en sous-cutané, la solution aqueuse homogène selon la présente invention va former avantageusement un système semi-cristallin, en particulier du fait du changement de pH lié à l'influence des milieux tamponnés de l'organisme.

Par « système semi-cristallin », on entend typiquement un système constitué d'une phase cristalline et d'une phase non cristalline (amorphe).

Typiquement, les cristaux de chitosane obtenus correspondent à l'allomorphe hydraté du chitosane.

De manière particulièrement avantageuse selon la présente invention, la solution aqueuse présente une bonne biocompatibilité et est biorésorbable. En particulier, le produit selon l'invention a une durée de biorésorption plus longue que les produits à base d'acide hyaluronique du type acide hyaluronique réticulé, pour un effet prolongé, tel qu'un effet de comblement prolongé.

Par « biorésorbable » ou « biorésorption », on entend une biodégradation qui aboutit à la dégradation totale ou essentiellement totale du produit injecté.

Selon une caractéristique particulière de la présente invention, la solution de chitosane est fluide avant injection et présente un temps de résorption long une fois injectée, typiquement de quelques semaines à plusieurs mois, par exemple de l'ordre de 3 ou 4 semaines jusqu'à 12 à 18 mois.

Le produit ou biomatériau constitué de ou contenant la solution aqueuse selon l'invention bénéficie du caractère bactério et fongistatique du chitosane, bien connu dans le monde de l'industrie agro-alimentaire et des pansements cicatrisants. Ces propriétés facilitent la conservation du produit et contribuent à limiter les risques d'infection liés à l'injection ou les phénomènes inflammatoires à retardement pour d'autres produits tels qu'évoqués ci-dessus. Face aux molécules naturelles utilisées à ce jour pour le comblement de rides (collagène, acide hyaluronique), le chitosane est le seul à présenter de telles propriétés.

Par ailleurs, le produit ou biomatériau constitué de ou contenant la solution aqueuse selon l'invention assure un comblement biologique efficace avantageusement immédiat: le chitosane favorisant en effet la synthèse de collagène permet un comblement des défauts cutanés, tels que les rides, par stimulation de mécanismes naturels.

La présente invention a également pour objet l'utilisation une solution aqueuse homogène de chitosane injectable contenant un chitosane ayant un degré d'acétylation inférieur à 20%, avantageusement inférieur à 10% et une masse moléculaire moyenne en masse comprise entre 100 000 et 1 000 000 g/mol, ladite solution contenant entre 0,1 et 3,5 %, avantageusement entre 1 et 2,5 %, en poids de chitosane, ladite solution présentant un pH inférieur à 6,2, avantageusement compris entre 5 et 6,2, ladite solution ne contenant pas de chitosane ayant un degré d'acétylation supérieur à 20% et ne contenant pas un autre chitosane de plus faible masse moléculaire moyenne pour former des particules cristallines de chitosane après injection.

Avantageusement, le chitosane est tel que défini précédemment.

En particulier, le chitosane a une masse moléculaire moyenne en masse comprise entre 100 000 et 1 000 000 g/mol, par exemple entre 250 000 et 1 000 000 g/mol, typiquement entre 250 000 et 500 000 g/mol.
Typiquement, ladite solution aqueuse ne contient pas de chitosane ayant un degré d'acétylation supérieur à 20%.

De manière avantageuse, ladite solution aqueuse est susceptible d'être préparée selon les étapes du procédé mentionné précédemment.

La présente invention a également pour objet une composition comprenant une solution aqueuse selon l'invention, et éventuellement un composé ou un excipent acceptable, tel qu'un composé ou excipient pour favoriser la cristallinité de la solution après injection.

Dans un mode de réalisation particulier, la composition selon l'invention comprend un sel tel que le chlorure de sodium, ou tout autre excipent acceptable avantageusement pour ajuster l'osmolarité de la composition. L'ajout d'un sel tel que le chlorure de sodium peut être intéressant pour obtenir une solution isotonique.

Selon une caractéristique particulière de la présente invention, la composition peut comprendre en outre au moins un composé ayant une activité thérapeutique reconnue. On peut citer à titre d'exemple un composé analgésique, un composé anesthésique local comme la lidocaïne, mépivacaïne, bupivacaïne ou ropivacaïne, un composé angiogénique, un vaccin, une hormone, ou encore un composé actif du type facteur de croissance ou oligosaccharide bioactif, par exemple un oligosaccharide d'acide hyaluronique, ou encore un acide nucléique ou une protéine.

Avantageusement selon la présente invention, la composition est formulée pour être administrée ou est utilisée par injection intradermique ou sous-cutanée.

La présente invention a également pour objet une telle composition ou une solution aqueuse selon l'invention pour son utilisation comme composition dermatologique, cosmétique, ou encore comme dispositif médical, avantageusement comme implant biorésorbable.

La présente invention a également pour objet l'utilisation cosmétique ou une méthode de traitement cosmétique ou esthétique du corps ou du visage humain comprenant l'injection d'une composition ou d'une solution aqueuse selon l'invention.

Dans un mode de réalisation particulier, la composition ou la solution aqueuse selon la présente invention est destinée à être utilisée dans la réparation ou la reconstruction des tissus de la peau du visage ou du corps.

En particulier, la composition ou la solution aqueuse selon la présente invention peut être utilisée pour le comblement des cavités du corps ou du visage, telles que les rides ou les ridules, pour la création ou l'augmentation de volumes du visage ou du corps humain, ou encore pour la cicatrisation de la peau.

Selon d'autres modes de réalisation particuliers, la composition ou la solution aqueuse selon la présente invention peut être utilisée :

- en chirurgie, notamment dans la réparation d'organes, ou en médecine ou chirurgie esthétique,
- en urologie, notamment pour le traitement de l'incontinence urinaire,
- en infectiologie, notamment comme fluide vecteur pour les vaccins,
- en ophtalmologie, notamment pour la cicatrisation cornéenne,
- en odontologie, notamment pour la pose d'un implant dentaire ou pour la réparation osseuse,
- ou encore en angiologie.

La composition ou la solution aqueuse selon la présente invention peut également être utilisée en rhumatologie.

Avantageusement, la composition ou la solution aqueuse selon la présente invention peut également être utilisée en tant que vecteur de principe actif notamment thérapeutique, tel qu'un vaccin ou une hormone du type insuline ou oestrogène, et d'une manière plus générale pour tous les principes actifs dont la délivrance ou libération contrôlée et/ou prolongée présente un avantage.

Enfin, la présente invention concerne l'utilisation cosmétique d'une solution aqueuse ou d'une composition selon l'invention pour traiter ou lutter contre le vieillissement de la peau.

Les exemples suivants sont destinés à illustrer l'invention sans aucunement en limiter la portée.

<u>Exemple 1 : Etude de la cristallisation des solutions de chitosane selon l'invention in vitro dans le PBS :</u>

Dans le cadre de cette expérimentation in vitro, le PBS (« Phosphate Buffer Saline » - solution saline de tampon phosphate) a été choisi pour simuler le milieu physiologique. Il s'agit en effet d'un milieu tampon isotonique de pH 7.2-7.4, couramment utilisé en biologie.

Les solutions testées sont des solutions de chitosane de différents degrés d'acétylation (DA), de concentration C=3% en poids de chitosane par rapport au poids de solution, et de pH compris entre 5 et 5.5.

Le chitosane utilisé est un chitosane issu de chitine de calamar (Mahtani Chitosan Veraval, Inde) et a une masse moléculaire moyenne en masse de 400 000 g/mol, évaluée grâce à un protocole décrit dans «Physico-chemical studies of the gelation of chitosan in a hydroalcoholic médium » A. MONTEMBAULT, C. VITON, A. DOMARD Biomaterials, 26(8), 933-943, 2005. Les solutions ont été préparées à l'aide d'acide acétique.

Les différentes solutions de chitosane testées ont un DA de 2%, 3,5%, 15%, 40%, 55%, toutes ces solutions présentant une teneur en chitosane de 3% en poids.

Ont également été testés un mélange de solutions de chitosane de DA 15% et 40%, de concentrations respectives 0,5 et 2% en poids de chitosane, ainsi qu'un mélange de solutions de chitosane de DA 15% et 55%, de concentrations respectives 0,5 et 2% en poids de chitosane, afin de comparer les produits selon l'invention et les produits tels que décrits dans les demandes de brevet de l'art antérieur WO 2008/072230 et WO 2009/150651.

Les différents DA sont obtenus par réacétylation d'un chitosane de calamar (Mahtani Chitosan Veraval, Inde) de DA 3,5%, Mw environ 400 000g.mol$^{-1}$, purifié par filtration d'une solution d'acétate de chitosane à la concentration 0,5% en poids en polymère par un filtre à 0,45$\mu$m. la solution est ensuite lyophilisée.

Dans un réacteur, et sous agitation mécanique (environ 50 tours par minute), le lyophilisat de chitosane est dissous dans de l'eau permutée à l'aide de la quantité stoechiométrique d'acide acétique nécessaire à la protonation des sites $NH_2$. Différentes concentrations (0,5w% à 3w%) ont été étudiées.

Le pH de chaque solution a été contrôlé, et est dans tous les cas compris entre 5 et 5,5 (en fonction de la concentration en chitosane).

0,30mL de chaque solution de chitosane a été introduit dans 30mL de PBS et laissé dans ce milieu pendant 24h et 72h. Les échantillons ont alors été retirés du PBS, placés dans des capillaires remplis d'eau pure stérile et analysés sous faisceau synchrotron en diffraction des rayons X, à l'aide d'un faisceau monochromatique à 16 keV ($\lambda$=0.7749 Å) sur la ligne D2AM, ESRF, Grenoble. L'intensité diffractée par les échantillons est donnée en fonction du vecteur de diffusion q= (4$\pi$ sin$\theta$)/$\lambda$, où 2$\theta$ est l'angle de diffraction (entre le faisceau incident et le faisceau diffracté) et après soustraction de l'intensité diffractée par le capillaire rempli d'eau seul, de manière à soustraire du mieux possible la contribution à la diffraction de l'eau et du contenant.

Les résultats de cette étude in vitro sont présentés sur les figures 1 à 3.

Les Figures la, 1b et 1c présentent les résultats de solutions de chitosane selon l'invention, avec un bas DA (inférieur ou égal à 15%).

La Figure la présente l'intensité diffractée par une solution de chitosane de DA 2%, C=3w%, après 24h dans un tampon PBS.

La Figure 1b présente l'intensité diffractée par une solution de chitosane de DA 3.5%, C=3w%, après 24h dans un tampon PBS.

La Figure 1c présente l'intensité diffractée par une solution de chitosane de DA 15%, C=3w%, après 24H dans un tampon PBS.

Les Figures 2a et 2b présentent les résultats de solutions de chitosane qui ne rentrent pas dans le cadre de la présente invention, avec un haut DA (de 40 à 55%), qui sont utilisés à titre de produits comparatifs avec les produits selon la présente invention.

La Figure 2a présente l'intensité diffractée par une solution de chitosane de DA 40%, C=3w%, après 24h dans un tampon PBS.

La Figure 2b présente l'intensité diffractée par une solution de chitosane de DA 55%, C=3w%, après 24h dans un tampon PBS.

Les Figures 3a et 3b présentent les résultats de solutions de chitosane qui ne rentrent pas dans le cadre de la présente invention, avec un mélange de bas DA et de haut DA, qui sont utilisés à titre de produits comparatifs avec les produits selon la présente invention.

La Figure 3a présente l'intensité diffractée par un mélange de solutions de chitosane de DA 15% et 40%, de concentrations respectives C=0.5w% et 2w%, après 72h dans un tampon PBS.

La Figure 3b présente l'intensité diffractée par un mélange de solutions de chitosane de DA 15% et 55%, de concentrations respectives C=0.5w% et 2w%, après 72h dans un tampon PBS.

On observe un pic de cristallinité représentatif de la raie (200) du chitosane hydraté (voir par exemple Osorio-Madrazo et al, Biomacromolecules 2010, 11, 1376-1386) vers 1,40Å$^{-1}$ après 24H dans le PBS pour une solution selon la présente invention de bas DA (2, 3.5% et 15%), de concentration 3% en poids (voir figures la, 1b et 1c).

En revanche pour les plus hauts DA (40% et 55%), qui ont été testés à titre comparatif avec l'invention, aucune cristallinité n'est observée à 24h ni même à 72H ; à des temps plus longs car les échantillons se solubilisent dans le milieu PBS et ne cristallisent pas (voir figures 2a et 2b).

Les solutions de bas DA selon l'invention, du fait de leur plus forte aptitude à cristalliser, peuvent donc être distinguées par un comportement en diffraction spécifique, malgré la très faible teneur en polymère des solutions qui sont constituées à plus de 97% d'eau.

Après un séjour dans le PBS, les solutions de bas DA deviennent donc des systèmes semi-cristallins. Les solutions de bas DA selon l'invention vont ainsi présenter un effet de comblement plus long grâce à la présence de cette cristallinité, tandis que les produits de haut DA vont avoir tendance à se solubiliser et se dégrader plus vite dans les tissus.

Les solutions constituées de chitosane de mélanges de DA telles que décrites dans les demandes de brevet de l'art antérieur WO 2008/072230 et WO 2009/150651 (voir les figures 3a et 3b qui montrent des résultats concernant

des systèmes de mélanges de DA 15% et 40% ; et 15% 55% respectivement) donnent des résultats similaires aux solutions contenant du chitosane seul de DA 40% ou 55% (voir figures 2a et 2b). Ces solutions ne cristallisent pas dans les conditions physiologiques et sont complètement solubilisées dans le milieu PBS au bout de 4 jours.

Exemple 2 : Etude in vivo : évaluation de performance et de la tolérance locale de solutions injectables de chitosane, implantées en intradermique chez le lapin:

L'objectif de la présente étude est l'évaluation de la tolérance locale macroscopique (par l'évaluation de l'érythème, oedème, nécrose et ulcération) et de la performance (selon les critères de dureté et diamètre) de 6 formulations test, en comparaison avec 3 produits de référence, après implantation intradermique chez le lapin.

Eléments d'essai :

Les compositions suivantes ont été testées : solutions aqueuses de chitosane, présentant une teneur de 3% en poids en mélange avec NaCl 9‰ + lidocaïne 0,3%, stérilisée par autoclave à 121°C pendant 15 minutes.
Les différents DA sont obtenus par réacétylation d'un chitosane de calamar (Mahtani Chitosan Veraval, Inde) de DA 3,5%, Mw environ 400 000g.$mol^{-1}$, purifié par filtration d'une solution d'acétate de chitosane à la concentration 0,5% en poids en polymère par un filtre à 0.45μm. la solution est ensuite lyophilisée.
Dans un réacteur, et sous agitation mécanique (50 tours par minute), le lyophilisat de chitosane est dissous dans de l'eau permutée à l'aide de la quantité stoechiométrique d'acide acétique nécessaire à la protonation des sites $NH_2$. Différentes concentrations (0,5w% à 3w%) ont été étudiées.
Le pH de chaque solution a été contrôlé, et est dans tous les cas compris entre 5 et 5,5 (en fonction de la concentration en chitosane).

Test 1: chitosane C=3w%, DA= 5%
Test 2: chitosane C=3w%, DA= 15%
Test 3: chitosane C=3w%, DA= 40%
Test 4: chitosane C=3w%, DA= 55%
Test 5: chitosane C=3w%, DA= 5% + 40%
Test 6: chitosane C=3w%, DA= 15% + 55%

Eléments de référence :

Réf 1: Restylane® Perlane Lidocaine (aiguille 29G) identifié sous la référence: ref 1-Restylane
Réf 2: Juvederm® Ultra 4 (aiguille 27G) identifié sous la référence: ref 2-Juvederm
Réf 3: New Fill® / Sculptra (aiguille 26G) identifié sous la référence : ref 3-NewFill.

Système d'essai :

Espèce : lapin
Souche : New Zealand White
Provenance : Charles River Laboratories
Statut sanitaire : IOPS (SPF)
Nombre d'animaux : 8 + 1 réserve
Sexe : femelle
Age à l'arrivée: 18 semaines

Implantations :

*Procédures préimplantatoires :*

A J0, les animaux ont été pesés, examinés puis anesthésiés, selon le protocole suivant:

- Kétamine (Ketamine 1000® - VIRBAC) 30 mg/kg , soit 0,3mL/kg

- + Médétomidine (Domitor® - Janssen Santé Animale) 0,1mg/kg, soit 0,1mL/kg
- Injection en intra-musculaire (IM) dans une cuisse.
- La zone dorsale a été tondue soigneusement (tondue à nouveau quand cela s'est avéré nécessaire par la suite pour les observations.)

*Procédure d'implantation :*

Six injections ont été réalisées par animal, sur la zone dorsale. On a veillé à ne pas injecter trop près de la zone de la nuque et des épaules, de façon à ce que la manipulation de l'animal n'entraîne pas de dégradation des sites.

Chaque site a été repéré par un tatouage, puis injecté avec 200μL de produit.

Suivi des animaux après implantation :

*Observations quotidiennes*

Elles ont été réalisées chaque jour par le personnel en charge des soins quotidiens (alimentation, abreuvement, nettoyage...).

Elles comprenaient une pesée de l'animal, un examen physique complet, et une observation comportementale rapide lors des manipulations.

*Examens cliniques approfondis*

Ils ont été réalisés par le vétérinaire sanitaire, le Directeur d'Etude ou son suppléant, généralement lorsqu'une anomalie conséquente a été constatée lors d'une observation quotidienne ou d'un examen clinique simple.

Ils incluaient une pesée, ainsi que la mesure des rythmes respiratoires et cardiaques, et la prise de la température rectale.

Les systèmes lymphatique, circulatoire, respiratoire, digestif, musculo-squelettique, nerveux, ainsi que la peau et les muqueuses ont été examinés.

*Observations macroscopiques*

Les observations ont eu lieu aux temps suivants:
J0 (post-implantation), T+24h, T+48h, J4.

- L'animal a été photographié vu de dessus, en s'assurant que le tatouage et l'ensemble des sites soient visibles.
- Les sites d'implantation ont été évalués visuellement ou manuellement grâce à une grille de scores.
- Le diamètre des sites a été mesuré au pied à coulisse.

Les paramètres évalués ont été : la formation d'oedème et d'érythème, les phénomènes d'ulcération et de nécrose localisés aux sites d'implantation, ainsi que la dureté et le diamètre des sites.

*Echelle de note pour oedème/érythème/ulcère/nécrose :*

(0) absent / (1) léger/ (2) modéré / (3) marqué / (4) sévère

Euthanasie et prélèvements :

A J2, cinq des huit animaux ont été anesthésiés, puis ont reçu une injection en intracardiaque de pentobarbital sodique (Dolethal® - VETOQUINOL).

Les sites d'implantation ont été prélevés, et placés en histocassettes identifiées.

Les prélèvements des lapins 1 à 3 ont été conservés dans du formol avant traitement pour étude histologique.

Les prélèvements des lapins 4 et 5 ont été conservés dans l'eau pure stérile. Quelques heures plus tard, on a procédé à l'extraction de l'implant dans les tissus, pour analyse sous faisceau synchrotron (technique WAXS, ESRF Grenoble, ligne D2AM). Un fragment d'explant est placé dans un capillaire rempli d'eau puis observé sous faisceau synchrotron en diffraction des rayons X, à l'aide d'un faisceau monochromatique à 16 keV ($\lambda$=0.7749 Å).

A J4, les trois animaux restants ont été anesthésiés, puis ont reçu une injection en intracardiaque de pentobarbital sodique (Dolethal® - VETOQUINOL).

Les sites d'implantation ont été prélevés, et placés en histocassettes identifiées. Ces prélèvements ont été

conservés dans du formol avant traitement pour étude histologique.

Résultats :

*Observations cliniques :*

Dans tous les cas, au cours des 4 premiers jours, l'oedème et l'érythème sont d'autant plus importants que le DA est élevé. Les solutions de DA 5% et 15% induisent chez certains animaux un oedème et un érythème scorés à 0, tandis que les solutions de DA≥40% induisent un oedème et un érythème scorés à 3 ou 4. Les solutions de DA 5% et 15% selon la présente invention sont les seules qui n'ont pas induit de nécrose systématique des sites implantés.
Il est important de noter que l'implant constitué de chitosane de DA55% n'est plus palpable après 4 jours, et qu'après 2 jours, il n'a pas été possible de le retirer des tissus pour l'analyse de diffraction X.
L'exemple de deux lapins (L4 et L5) ayant reçu les formulations « test » est présenté sur les figures 4a et 4b.
Les figures 4a et 4b montrent la photographie des sites d'injection respectivement des lapins 4 et 5, 24h après l'implantation :

A : Test 1 : chitosane C=3w%, DA= 5%
B : Test 2 : chitosane C=3w%, DA= 15%
C : Test 3 : chitosane C=3w%, DA= 40%
D : Test 4 : chitosane C=3w%, DA= 55%
E : Test 5 : chitosane C=3w%, DA= 5% + 40%
F : Test 6 : chitosane C=3w%, DA= 15% + 55%

Les solutions préparées selon la présente invention induisent une réponse inflammatoire limitée par rapport aux solutions contenant des chitosanes de haut DA ou des mélanges de chitosanes contenant notamment un chitosane de haut DA telles que décrites dans demandes de brevet WO 2008/072230 et WO 2009/150651. De plus, comme le suggère la complète disparition de l'implant de degré d'acétylation égal à 55% après seulement 4 jours, l'utilisation d'un chitosane de haut DA ne confère pas une durée de biorésorption satisfaisante pour les applications visées.

Etude des explants sous faisceau synchrotron

Les figures 5 à 7 représentent les résultats de l'étude sous faisceau synchrotron en diffraction des rayons X d'explants de chitosane 24h après l'implantation des solutions en intradermique.
Les Figures 5a et 5b représentent les résultats d'une solution de chitosane selon l'invention (site A, test 1), avec un bas DA égal à 5%, après l'implantation de la solution respectivement chez le Lapin 4 et le Lapin 5.
Les Figures 5c et 5d représentent les résultats d'une solution de chitosane selon l'invention (site B, test 2), avec un bas DA égal à 15%, après l'implantation de la solution respectivement chez le Lapin 4 et le Lapin 5.
Les Figures 6a et 6b représentent les résultats d'une solution de chitosane comparative (site C, test 3), avec un haut DA égal à 40%, après l'implantation de la solution respectivement chez le Lapin 4 et le Lapin 5.
Les Figures 7a et 7b représentent les résultats d'une solution de chitosane comparative (site E, test 5), avec un mélange de bas DA et de haut DA : 5 + 40%, après l'implantation de la solution respectivement chez le Lapin 4 et le Lapin 5.
Les Figures 7c et 7d représentent les résultats d'une solution de chitosane comparative (site F, test 6), avec un mélange de bas DA et de haut DA : 15 + 55%, après l'implantation de la solution respectivement chez le Lapin 4 et le Lapin 5.

L'intensité diffractée par les explants est donnée en fonction du vecteur de diffusion $q = (4\pi \sin\theta)/\lambda$, où $2\theta$ est l'angle de diffraction (entre le faisceau incident et le faisceau diffracté) et après soustraction de l'intensité diffractée par le capillaire rempli d'eau seul, de manière à soustraire du mieux possible la contribution à la diffraction de l'eau et du contenant.
On observe, en plus du résidu de halo amorphe dû à l'eau, un pic de cristallinité bien défini représentatif de la raie 200 du chitosane hydraté vers $1.40Å^{-1}$ pour les explants de bas DA (5% et 15%), tandis que celui-ci est très peu perceptible (DA 40%, mélange DA 5%+40%) ou complètement absent (DA 15%+55%). Les solutions de bas DA, du fait de leur aptitude à cristalliser plus forte, peuvent donc être distinguées par un comportement en diffraction spécifique, malgré la très faible teneur en polymère des solutions qui sont constituées à plus de 97% d'eau.
Après injection dans le derme, ces solutions de bas DA deviennent donc des systèmes semi-cristallins. On

peut ainsi s'attendre à un effet de comblement plus long grâce à la présence de cette cristallinité, tandis que les produits de haut DA auront tendance à se solubiliser et se dégrader plus vite dans les tissus.

Les solutions constituées de chitosane de mélanges de DA telles que décrites dans les demandes de brevet de l'art antérieur WO 2008/072230 et WO 2009/150651 donnent des résultats similaires aux solutions contenant du chitosane seul de DA 40% ou 55% : ces solutions ne cristallisent pas ou très peu dans les tissus et, pour le DA 55%, elles ne sont macroscopiquement plus observables sous quatre jours.

La cristallinité développée in situ permet en effet d'allonger le temps de biorésorption, et est donc de grand intérêt pour les applications visées.

Exemple 3 : Etude in vivo : évaluation de performance et de la tolérance locale de solutions injectables de chitosane, implantées en sous-cutané chez le rat:

L'objectif de la présente étude est l'évaluation de la tolérance locale macroscopique (par l'évaluation de l'érythème, oedème, nécrose et ulcération) et de la performance (selon les critères de dureté) de 2 formulations test, en comparaison avec 1 produit de référence, après injection sous-cutanée chez le rat.

Eléments d'essai :

Les compositions suivantes ont été testées : solutions aqueuses de chitosane, présentant une teneur de 3% en poids en mélange avec NaCl 9‰ + lidocaïne 0,3%, stérilisée par autoclave à 121°C pendant 15 minutes.

Le chitosane utilisé est un chitosane de calamar (Mahtani Chitosan Veraval, Inde) de DA 2%, Mw (masse molaire) environ 400 000 $g.mol^{-1}$, purifié par filtration d'une solution d'acétate de chitosane à la concentration 0,5% en poids en polymère par un filtre à 0.45$\mu$m. La solution est ensuite lyophilisée.

Dans un réacteur, et sous agitation mécanique (50 tours par minute), le lyophilisat de chitosane est dissout dans de l'eau pour préparation injectable à l'aide de la quantité stoechiométrique d'acide acétique nécessaire à la protonation des sites $NH_2$. La concentration 3w% a été étudiée.

Le pH de chaque solution a été contrôlé, et est dans tous les cas compris entre 5 et 6,2.

Test 1: chitosane C=3w%, DA= 2%, pH = 5.
Test 2: chitosane C=3w%, DA= 2%, pH = 6.

Eléments de référence :

Réf 1: Restylane® Perlane Lidocaine (aiguille 29G)

Système d'essai :

Espèce : rat
Souche : Sprague Dawley
Nombre d'animaux : 6
Sexe : mâle
Age à l'arrivée: entre 7 et 8 semaines
Poids à l'arrivée : entre 200 et 220 grammes

Implantations :

*Procédures préimplantatoires :*

A J0, les animaux ont été pesés, examinés puis anesthésiés, selon le protocole suivant:
Injection intrapéritonéale (1 mL/100 g de poids d'animal) d'une dilution de Pentobarbital sodique (CEVA SANTE ANIMALE - 100 mL à 54,7 mg/mL) à raison de 6 mL pour 44 mL de sérum physiologique.

La zone dorsale a été tondue soigneusement (tondue à nouveau lorsque cela s'est avéré nécessaire).

Aucun traitement antibiotique n'a été mis en place.

*Procédure d'implantation :*

Quatre injections par voie sous-cutanée à l'aide de seringues en verre stériles avec aiguille stérile ont été réalisées par animal, sur la zone dorsale.
Chaque site d'implantation a été repéré par tatouage, puis injecté avec 100µL de produit.
Les sites d'injection ont été randomisés, avec pour critère que chaque animal reçoive au moins une injection par formulation (test 1, test 2 et Réf 1).

Suivi des animaux après implantation :

*Observations quotidiennes*

Elles ont été réalisées chaque jour par le personnel en charge des soins quotidiens (alimentation, abreuvement, nettoyage...).
Elles comprenaient une pesée de l'animal, un examen physique complet, et une observation comportementale rapide lors des manipulations.

*Observations macroscopiques*

Les observations ont eu lieu aux temps suivants:
J0 (post-implantation), J2 (T+48h), J4 (T+96h).
Les sites d'implantation ont été évalués visuellement ou manuellement grâce à une grille de scores.
Les paramètres évalués ont été : la formation d'oedème et d'érythème, les phénomènes d'ulcération et de nécrose localisés aux sites d'implantation, ainsi que la dureté.

*Echelle de note pour oedème/érythème/ulcère/nécrose :*

(0) absent / (1) léger/ (2) modéré / (3) marqué / (4) sévère

Euthanasie et prélèvements :

A J4, les animaux ont été anesthésiés, puis ont reçu une injection Pentobarbital sodique (2 mL non dilués en IP). Les sites d'implantation ont été prélevés de manière à couvrir la lésion et une zone non lésée attenante, chaque prélèvement comprenant toutes les couches de la peau jusqu'au muscle. Les prélèvements ont été fixés dans une solution aqueuse de formaldéhyde à 4% pendant 48h.

Résultats :

*Observations cliniques :*

Tous les animaux ont présenté un aspect et un comportement normaux durant toute la période d'observation et leur poids est resté stable.
L'évaluation visuelle et manuelle des sites implantés n'a révélé aucune différence liée aux formulations testées.
Il n'a pas été observé d'érythèmes induits par les solutions selon la présente invention injectées par voie sous-cutanée.
Les volumes observés ne sont pas liés à un effet irritant des solutions testées mais sont d'origine mécanique (implant non résorbé).
Dans les conditions expérimentales adoptées, les 2 formulations de solution selon la présente invention ont été bien tolérées localement.
L'évaluation de la tolérance locale macroscopique et de la performance des formulations test 1 et test 2 a été comparable à celle de la formulation de référence Réf 1.
Aucune réaction adverse significative telle que nécrose importante et ulcération de la peau n'a été observée. Les solutions préparées selon la présente invention induisent donc une réponse inflammatoire limitée.

*Examen histologique des implants*

Les échantillons ont été fixés au moins 24h avant de pouvoir être déshydratés.
Une coupe (épaisseur 3 à 5 µm) a été effectuée par bloc. Les lames ont été colorées à l'hématoxyline-éosine.

Vingt-quatre lames virtuelles ont été analysées.

L'aspect histologique des implants est très différent entre l'implant de référence (Restylane/Perlane/Lidocaïne) et les implants testés (test 1 et test 2). Alors que l'implant de référence était homogène, les implants testés présentaient un aspect soit micro-globulaires (diamètre variable, le plus souvent entre 5 et 15 μm - Test1), soit de type coagulum (Test 2).

La réaction de l'hôte aux formulations test 1 et test 2 était le plus souvent limitée à l'hypoderme sous le muscle peaucier, consistant en une fibroplasie / tissu de granulation et inflammation légère à modérée entourant l'implant. Cette réaction consistait en un tissu de granulation riche en fibres de collagène en voie de maturation et une infiltration par des cellules mononucléées consistant en grande majorité à des monocytes / histiocytes et des lymphocytes, avec occasionnellement des plasmocytes, mais la plupart du temps sans granulocytes.

Basés sur ces critères, une classification de faible réaction de l'hôte (score 1) a été observée pour l'article de référence, une réaction intermédiaire (score 2) pour les formulations Test 1 et Test 2, avec cependant une réaction de l'hôte plus marquée dans le cas de l'article Test1.

Les solutions constituées de chitosane selon l'invention donnent des résultats similaires à la solution de référence en termes de tolérance. En revanche, tout comme dans l'exemple 2, après injection sous-cutanée, ces solutions de bas DA deviennent des systèmes semi-cristallins. On peut ainsi s'attendre à un effet de comblement plus long grâce à la présence de cette cristallinité.

## Revendications

1. Solution aqueuse homogène de chitosane injectable contenant un chitosane ayant un degré d'acétylation inférieur à 20%, avantageusement inférieur à 10% et une masse moléculaire moyenne en masse comprise entre 100 000 et 1 000 000 g/mol, ladite solution contenant entre 0,1 et 3,5 %, avantageusement entre 1 et 2,5 %, en poids de chitosane, ladite solution présentant un pH inférieur à 6,2, avantageusement compris entre 5 et 6,2, ladite solution ne contenant pas de chitosane ayant un degré d'acétylation supérieur à 20%, ladite solution ne contenant pas un autre chitosane de plus faible masse moléculaire moyenne et ladite solution aqueuse étant apte à former des particules cristallines de chitosane après injection.

2. Solution aqueuse selon la revendication 1, **caractérisée en ce que** le chitosane a une masse moléculaire moyenne en masse comprise entre 250 000 et 1 000 000 g/mol.

3. Solution aqueuse selon l'une quelconque des revendications précédentes, **caractérisée en ce qu'**elle est susceptible d'être préparée par les étapes suivantes :

   - dissolution du chitosane dans l'eau par ajout d'acide tel qu'un acide faible, ledit acide faible étant avantageusement choisi dans le groupe constitué par l'acide acétique, l'acide glycolique, l'acide lactique, l'acide glutamique, et leurs mélanges,
   - et éventuellement réajustement du pH pour obtenir une solution aqueuse présentant un pH compris entre 5 et 6,2.

4. Solution aqueuse selon la revendication 3, **caractérisée en ce que**, lors de l'étape de dissolution, l'acide est ajouté en une quantité strictement nécessaire à la dissolution du chitosane.

5. Composition comprenant une solution aqueuse selon l'une quelconque des revendications précédentes, et éventuellement un composé ou un excipent acceptable, tel qu'un composé ou excipient pour favoriser la cristallinité de la solution après injection, par exemple une chaîne courte de chitosane de degré d'acétylation inférieur à 20% et de masse moléculaire moyenne en masse inférieure à 20 OOOg/mol ou un chito-oligosaccharide de degré de polymérisation compris entre 3 et 30.

6. Composition selon la revendication 5, comprenant en outre au moins un composé actif tel qu'un composé analgésique, anesthésique local, tel que la lidocaïne, mépivacaïne, bupivacaïne ou ropivacaïne, un composé angiogénique, un vaccin, ou encore un composé actif du type facteur de croissance ou oligosaccharide bioactif.

7. Composition selon la revendication 5 ou 6, **caractérisée en ce qu'**elle est formulée pour être administrée par injection intradermique ou sous-cutanée.

8. Composition selon l'une quelconque des revendications 5 à 7, pour son utilisation comme composition dermatologique, cosmétique, ou encore comme dispositif médical, avantageusement comme implant biorésorbable.

**9.** Composition selon la revendication 8, pour son utilisation dans la réparation ou la reconstruction des tissus de la peau du visage ou du corps.

**10.** Composition selon la revendication 8 ou 9, pour son utilisation pour le comblement des cavités du visage, telles que les rides ou les ridules, pour la création ou l'augmentation de volumes du visage ou du corps humain, ou encore pour la cicatrisation de la peau.

**11.** Composition selon la revendication 8, pour son utilisation en chirurgie, en médecine ou chirurgie esthétique, en urologie, rhumatologie, ophtalmologie, odontologie, ou encore en angiologie.

**12.** Composition selon la revendication 8, pour son utilisation en tant que vecteur de principes actifs, par exemple comme véhicule de vaccins ou d'hormones.

**13.** Utilisation cosmétique d'une solution aqueuse selon l'une quelconque des revendications 1 à 4 ou d'une composition selon la revendication 5 pour traiter ou lutter contre le vieillissement de la peau.

**14.** Utilisation cosmétique d'une solution aqueuse homogène de chitosane injectable contenant un chitosane ayant un degré d'acétylation inférieur à 20%, avantageusement inférieur à 10% et une masse moléculaire moyenne en masse comprise entre 100 000 et 1 000 000 g/mol, ladite solution contenant entre 0,1 et 3,5 %, avantageusement entre 1 et 2,5 %, en poids de chitosane, ladite solution présentant un pH inférieur à 6,2, avantageusement compris entre 5 et 6,2, ladite solution ne contenant pas de chitosane ayant un degré d'acétylation supérieur à 20% et ne contenant pas un autre chitosane de plus faible masse moléculaire moyenne
pour former des particules cristallines de chitosane après injection.

**15.** Utilisation cosmétique selon la revendication 14, **caractérisée en ce que** le chitosane a une masse moléculaire moyenne en masse comprise entre 250 000 et 1 000 000 g/mol.

**Patentansprüche**

**1.** Injizierbare homogene wässrige Lösung eines Chitosans, umfassend ein Chitosan mit einem Acetylierungsgrad von weniger als 20%, vorzugsweise weniger als 10%, und einem gewichtsmittleren Molekulargewicht zwischen 100 000 und 1 000 000 g/mol, wobei die Lösung zwischen 0,1 und 3,5 Gewichts-%, vorzugsweise zwischen 1 und 2,5 Gewichts-% Chitosan umfasst, wobei die Lösung einen pH-Wert von weniger als 6,2, vorzugsweise zwischen 5 und 6,2 aufweist, wobei die Lösung kein Chitosan mit einem Acetylierungsgrad von mehr als 20% umfasst, wobei die Lösung kein anderes Chitosan mit einem niedrigeren mittleren Molekulargewicht umfasst und wobei die wässrige Lösung in der Lage ist, nach einer Injektion kristalline Chitosanpartikel zu bilden.

**2.** Wässrige Lösung nach Anspruch 1, **dadurch gekennzeichnet, dass** das Chitosan ein gewichtsmittleres Molekulargewicht zwischen 250 000 und 1 000 000 g/mol aufweist.

**3.** Wässrige Lösung nach einem der vorhergehenden Ansprüche, **dadurch gekennzeichnet, dass** sie durch die folgenden Schritte hergestellt werden kann:

- Auflösen von Chitosan in Wasser durch Zugabe einer Säure wie einer schwachen Säure, wobei die schwache Säure vorzugsweise ausgewählt ist aus der Gruppe bestehend aus Essigsäure, Glykolsäure, Milchsäure, Glutaminsäure und Mischungen davon,
- und gegebenenfalls erneutes Einstellen des pH-Werts, um eine wässrige Lösung zu erhalten, die einen pH-Wert zwischen 5 und 6,2 aufweist.

**4.** Wässrige Lösung nach Anspruch 3, **dadurch gekennzeichnet, dass** die Säure während des Auflösungsschrittes in einer Menge zugegeben wird, die für das Auflösen von Chitosan unbedingt erforderlich ist.

**5.** Zusammensetzung, umfassend eine wässrige Lösung nach einem der vorhergehenden Ansprüche und gegebenenfalls eine akzeptable Verbindung oder einen akzeptablen Hilfsstoff, wie eine Verbindung oder einen Hilfsstoff zur Förderung der Kristallinität der Lösung nach einer Injektion, zum Beispiel ein kurzkettiges Chitosan mit einem

Acetylierungsgrad von weniger als 20% und einem gewichtsmittleren Molekulargewicht von weniger als 20 000 g/mol oder ein Chitooligosaccharid mit einem Polymerisationsgrad zwischen 3 und 30.

6. Zusammensetzung nach Anspruch 5, ferner umfassend mindestens eine aktive Verbindung, wie eine analgetische Verbindung, ein Lokalanästhetikum, wie Lidocain, Mepivacain, Bupivacain oder Ropivacain, eine angiogene Verbindung, einen Impfstoff oder auch eine aktive Verbindung vom Typ Wachstumsfaktor oder bioaktives Oligosaccharid.

7. Zusammensetzung nach Anspruch 5 oder 6, **dadurch gekennzeichnet, dass** sie zur Verabreichung durch intradermale oder subkutane Injektion formuliert ist.

8. Zusammensetzung nach einem der Ansprüche 5 bis 7 zur Verwendung als dermatologische oder kosmetische Zusammensetzung oder auch als Medizinprodukt, vorzugsweise als bioresorbierbares Implantat.

9. Zusammensetzung nach Anspruch 8 zur Verwendung beim Reparieren oder Rekonstruieren von Gesichts- oder Körperhautgeweben.

10. Zusammensetzung nach Anspruch 8 oder 9 zur Verwendung zum Füllen von Gesichtshohlräumen, wie Falten oder Fältchen, zur Erzeugung oder Vergrößerung von Volumen des Gesichts oder des menschlichen Körpers oder auch zur Vernarbung der Haut.

11. Zusammensetzung nach Anspruch 8 zur Verwendung in der Chirurgie, in der Medizin oder Schönheitschirurgie, in der Urologie, Rheumatologie, Ophthalmologie, Odontologie oder auch in der Angiologie.

12. Zusammensetzung nach Anspruch 8 zur Verwendung als Vektor von Wirkstoffen, zum Beispiel als Vehikel von Impfstoffen oder Hormonen.

13. Kosmetische Verwendung einer wässrigen Lösung nach einem der Ansprüche 1 bis 4 oder einer Zusammensetzung nach Anspruch 5 zum Behandeln oder Bekämpfen der Hautalterung.

14. Kosmetische Verwendung einer injizierbaren homogenen wässerigen Lösung eines Chitosans umfassend Chitosan mit einem Acetylierungsgrad von weniger als 20%, vorzugsweise weniger als 10%, und einem gewichtsmittleren Molekulargewicht zwischen 100 000 und 1 000 000 g/mol, wobei die Lösung zwischen 0,1 und 3,5 Gewichts-%, vorzugsweise zwischen 1 und 2,5 Gewichts-% Chitosan umfasst, wobei die Lösung einen pH-Wert von weniger als 6,2, vorzugsweise zwischen 5 und 6,2 aufweist, wobei die Lösung kein Chitosan mit einem Acetylierungsgrad von mehr als 20% umfasst und kein anderes Chitosan mit einem niedrigeren mittleren Molekulargewicht umfasst um nach einer Injektion kristalline Chitosanpartikel zu bilden.

15. Kosmetische Verwendung nach Anspruch 14, **dadurch gekennzeichnet, dass** das Chitosan ein gewichtsmittleres Molekulargewicht zwischen 250 000 und 1 000 000 g/mol aufweist.

## Claims

1. An injectable homogeneous aqueous solution of chitosan containing a chitosan having a degree of acetylation lower than 20%, advantageously lower than 10%, and a mean molecular weight between 100,000 and 1,000,000 g/mol, said solution containing between 0.1 and 3.5% by weight, advantageously between 1 and 2.5% by weight of chitosan, said solution having a pH lower than 6.2, advantageously between 5 and 6.2, said solution not containing a chitosan having a degree of acetylation higher than 20%, said solution not containing another chitosan of lower mean molecular weight and said aqueous solution being capable of forming crystalline particles of chitosan after injection.

2. The aqueous solution according to claim 1, wherein the chitosan has a mean molecular weight between 250,000 and 1,000,000 g/mol.

3. The aqueous solution according to any of the preceding claims, wherein the solution can be prepared by the following steps:

- dissolution of the chitosan in water by the addition of an acid such as a weak acid, said weak acid being

advantageously selected from the group comprised of acetic acid, glycolic acid, lactic acid, glutamic acid, and mixtures thereof,
- and, optionally, readjustment of the pH in order to obtain an aqueous solution having a pH between 5 and 6.2.

**4.** The aqueous solution according to claim 3, wherein, during the dissolution step, the acid is added in an amount strictly necessary to dissolve the chitosan.

**5.** A composition comprising an aqueous solution according to any one of the preceding claims, and optionally an acceptable compound or excipient, such as a compound or excipient to promote the crystallinity of the solution after injection, for example a short-chain chitosan of degree of acetylation lower than 20% and mean molecular weight lower than 20,000 g/mol, or a chito-oligosaccharide of degree of polymerization between 3 and 30.

**6.** The composition according to claim 5, further comprising at least an active compound such as an analgesic compound, local anesthetic, such as lidocaine, mepivacaine, bupivacaine or ropivacaine, an angiogenic compound, a vaccine, or an active compound of the growth factor or bioactive oligosaccharide type.

**7.** The composition according to claim 5 or 6, **characterized in that** it is formulated to be administered by intradermal or subcutaneous injection.

**8.** The composition according to any one of claims 5 to 7, for use as a dermatological or cosmetic composition, or as a medical device, advantageously as a bioresorbable implant.

**9.** The composition according to claim 8, for use in the repair or reconstruction of tissues of the skin of the face or body.

**10.** The composition according to claim 8 or 9, for use in the filling of facial cavities such as lines or wrinkles, for creating or increasing the volume of the human face or body, or for the cicatrization of the skin.

**11.** The composition according to claim 8, for use in surgery, in cosmetic medicine or surgery, in urology, rheumatology, ophthalmology, odontology, or in angiology.

**12.** The composition according to claim 8, for use as a vector for active ingredients, for example as a carrier of vaccines or hormones.

**13.** A cosmetic use of an aqueous solution according to any one of claims 1 to 4 or of a composition according to claim 5 to treat or prevent skin aging.

**14.** Use of an injectable homogeneous aqueous solution of chitosan containing a chitosan having a degree of acetylation lower than 20%, advantageously lower than 10%, and a mean molecular weight between 100,000 and 1,000,000 g/mol, said solution containing between 0.1 and 3.5%, advantageously between 1 and 2.5%, by weight of chitosan, said solution having a pH lower than 6.2, advantageously between 5 and 6.2, said solution not containing a chitosan having a degree of acetylation higher than 20%, and not containing another chitosan of lower mean molecular weight to form crystalline particles of chitosan after injection

**15.** Use according to claim 14, **characterized in that** the chitosan has a mean molecular weight between 250,000 and 1,000,000 g/mol.

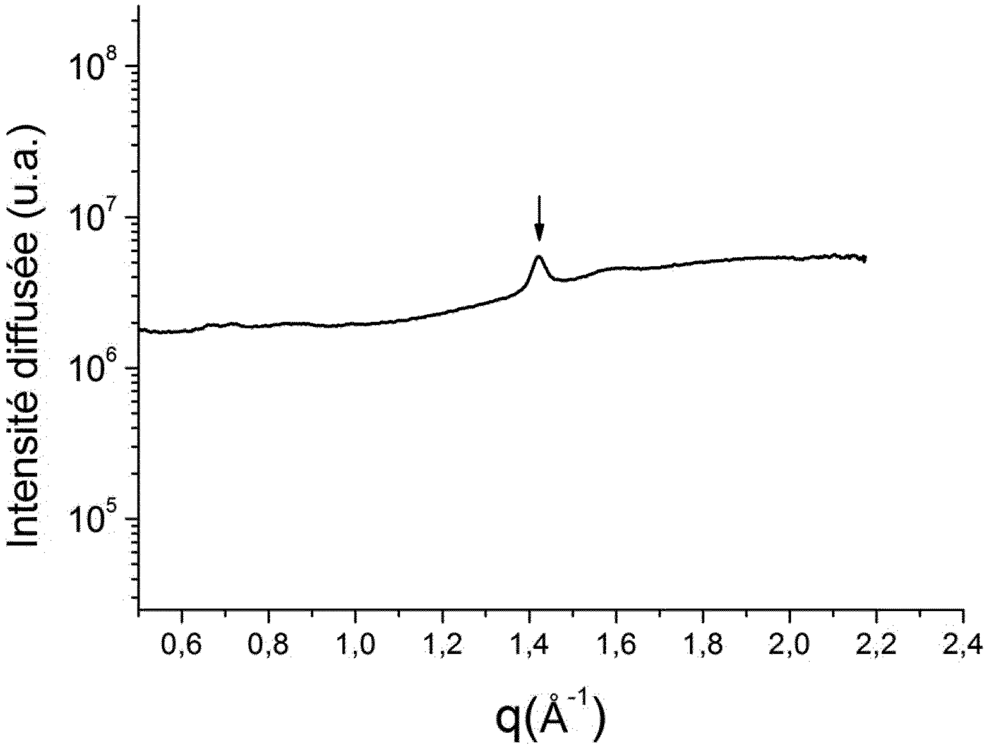


Figure 1a

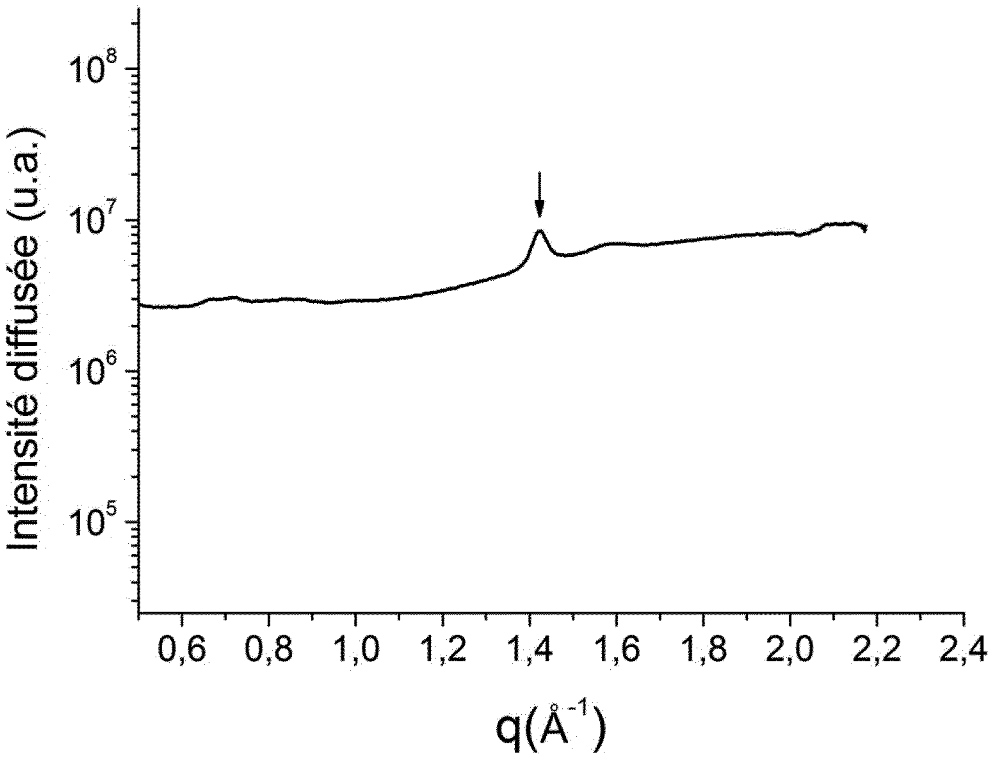


Figure 1b

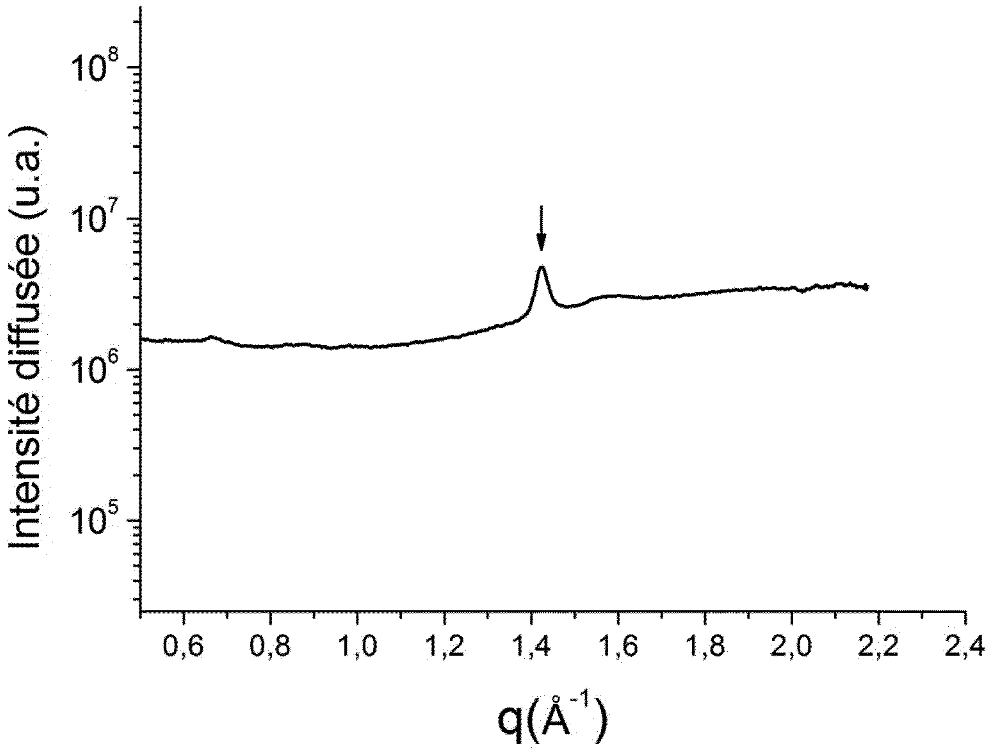
Intensité diffusée (u.a.)
10^8
10^7
10^6
10^5
0,6
0,8
1,0
1,2
1,4
1,6
1,8
2,0
2,2
2,4
q(Å^-1)

Figure 1c

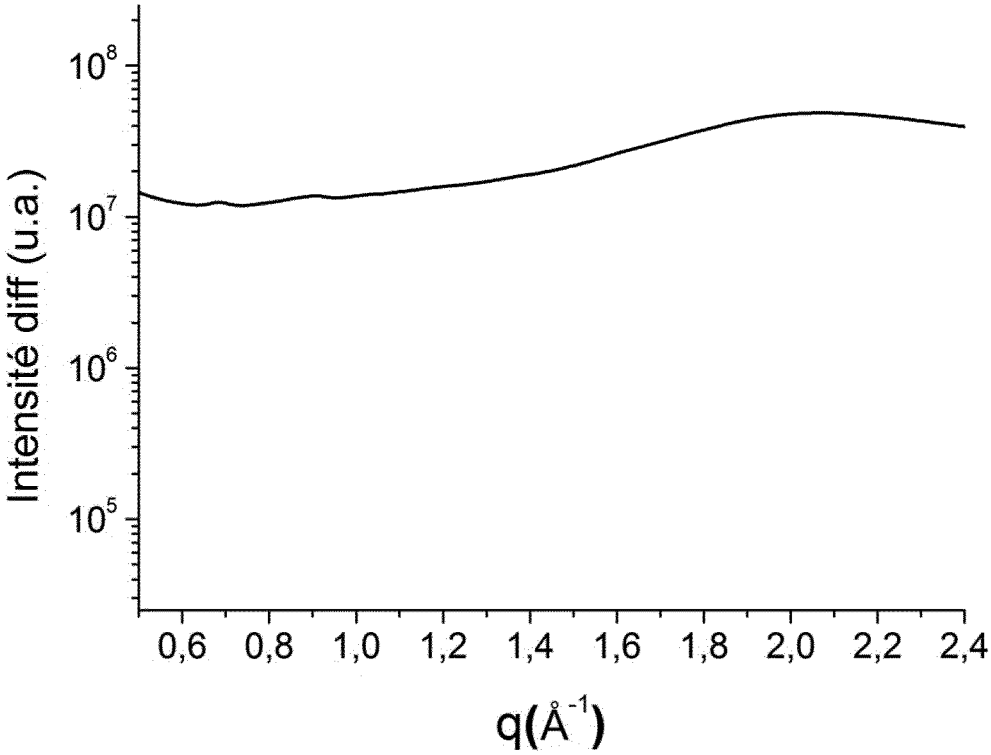


Figure 2a

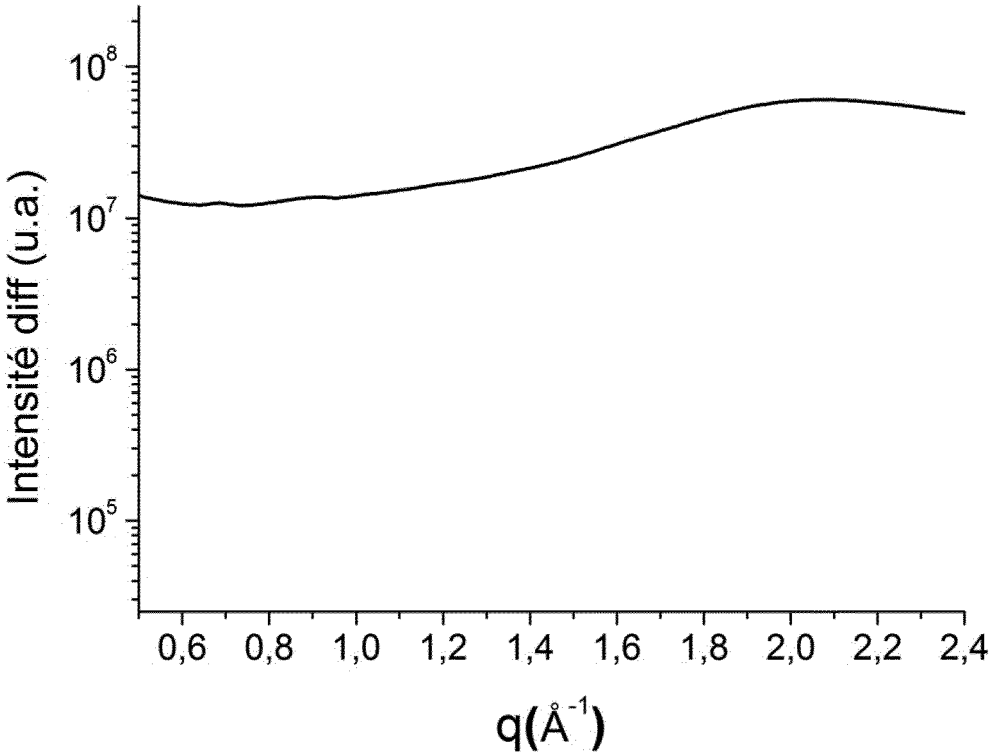


Figure 2b

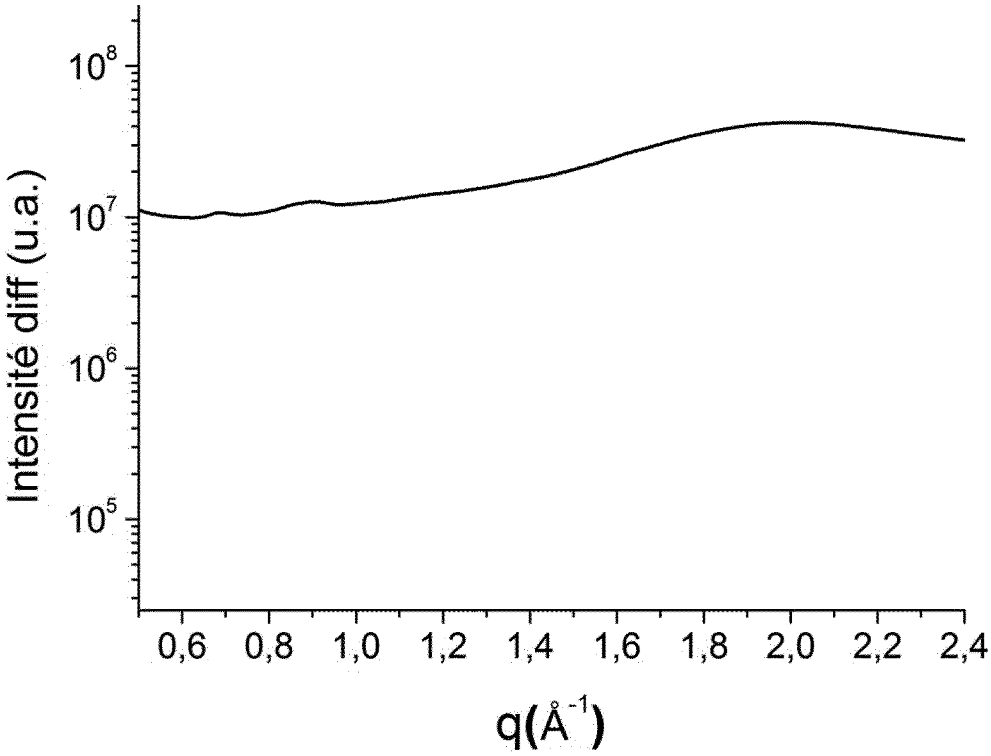


Figure 3a

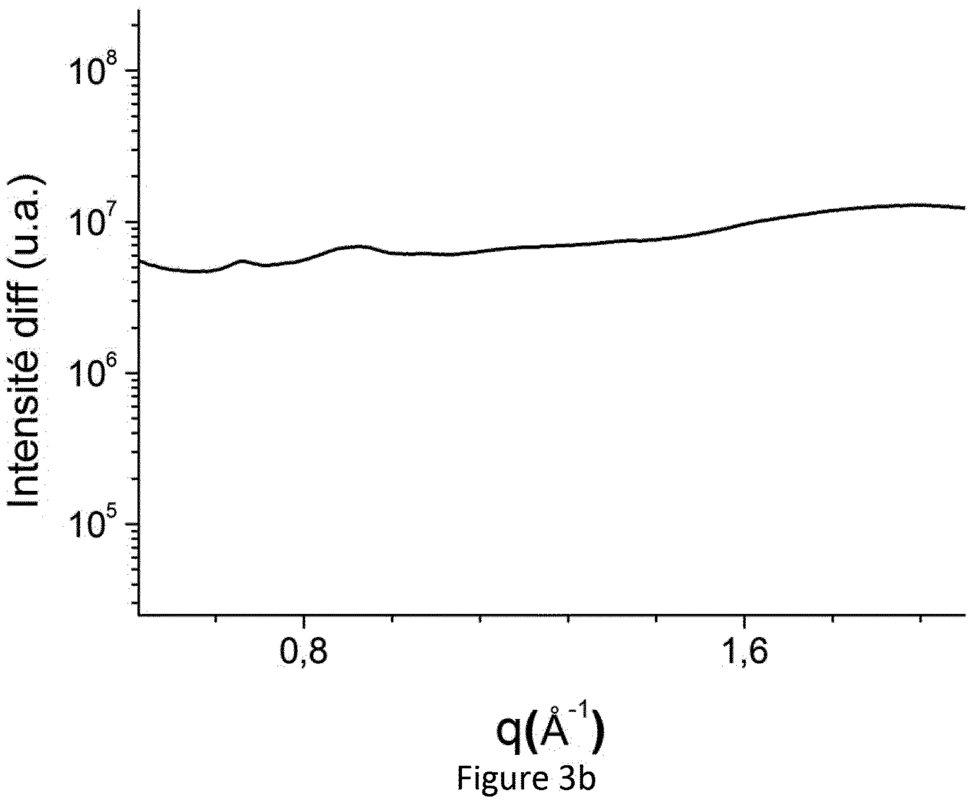


Figure 3b

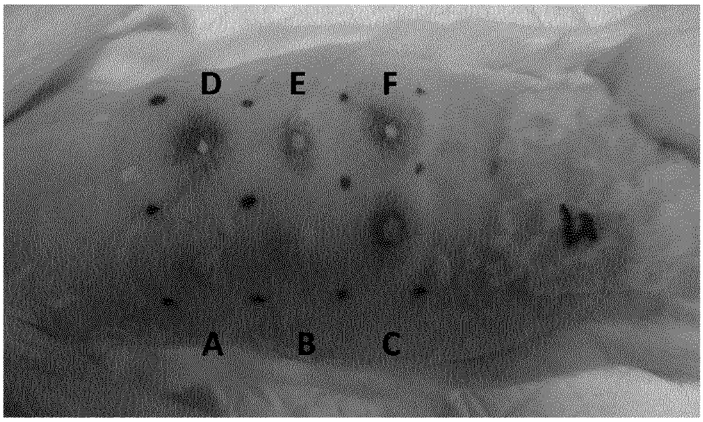


Figure 4a

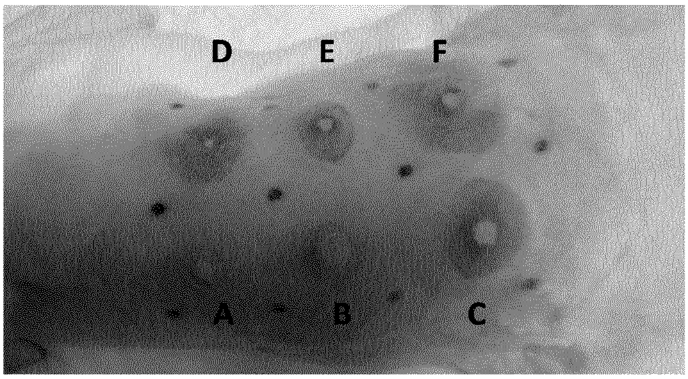


Figure 4b

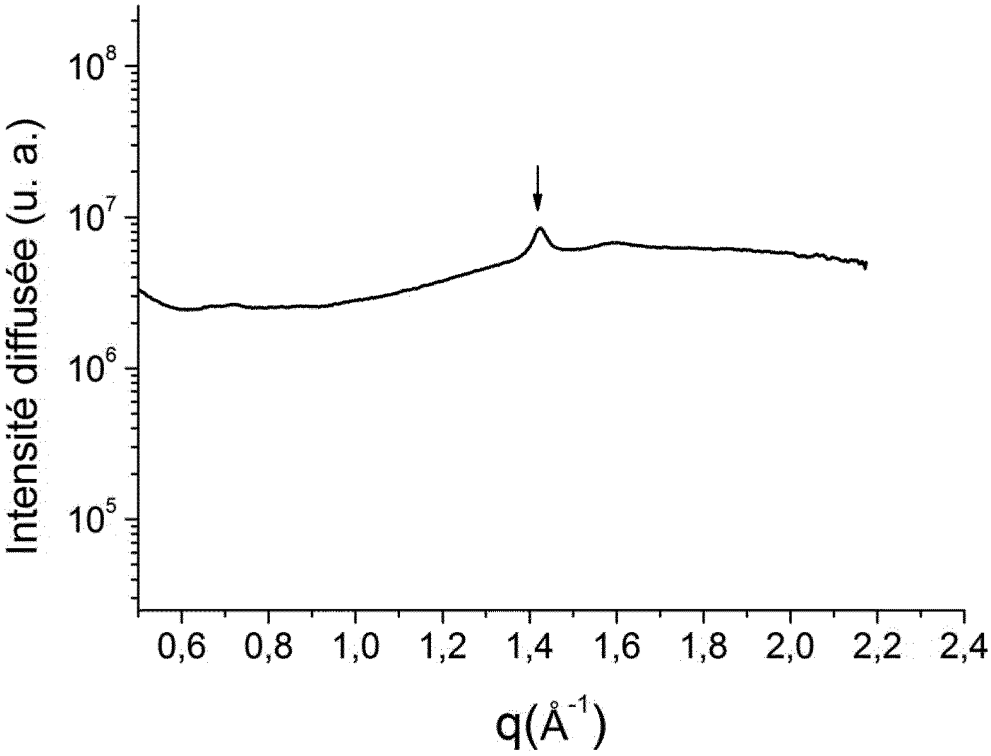


Figure 5a

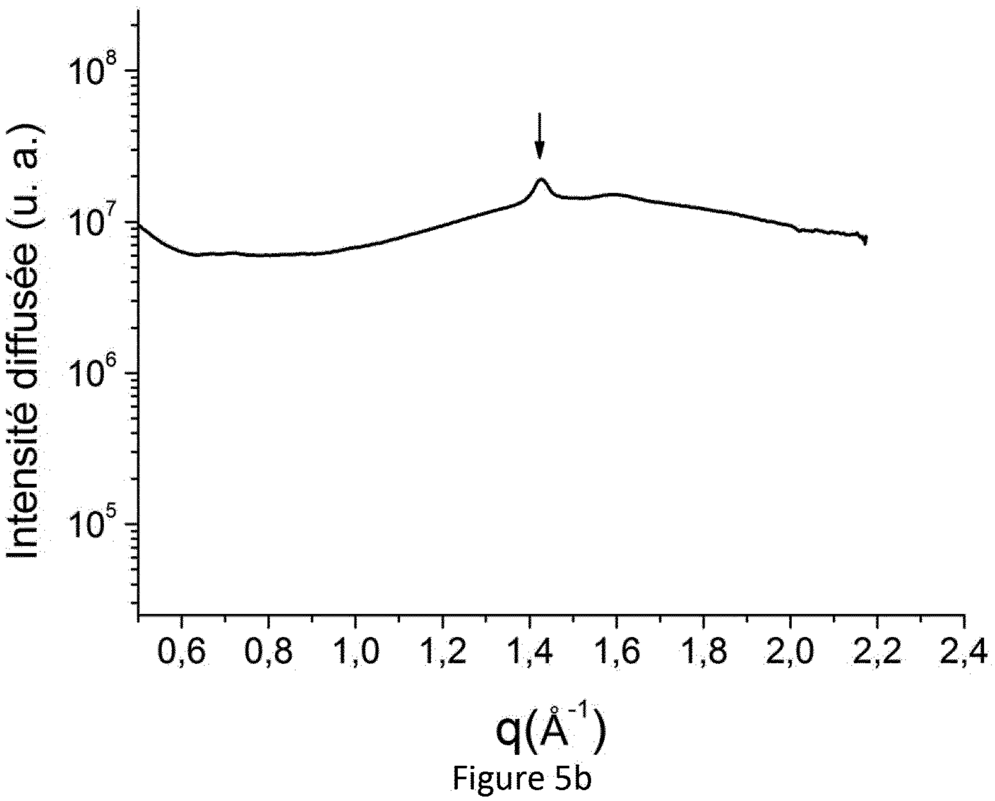


Figure 5b

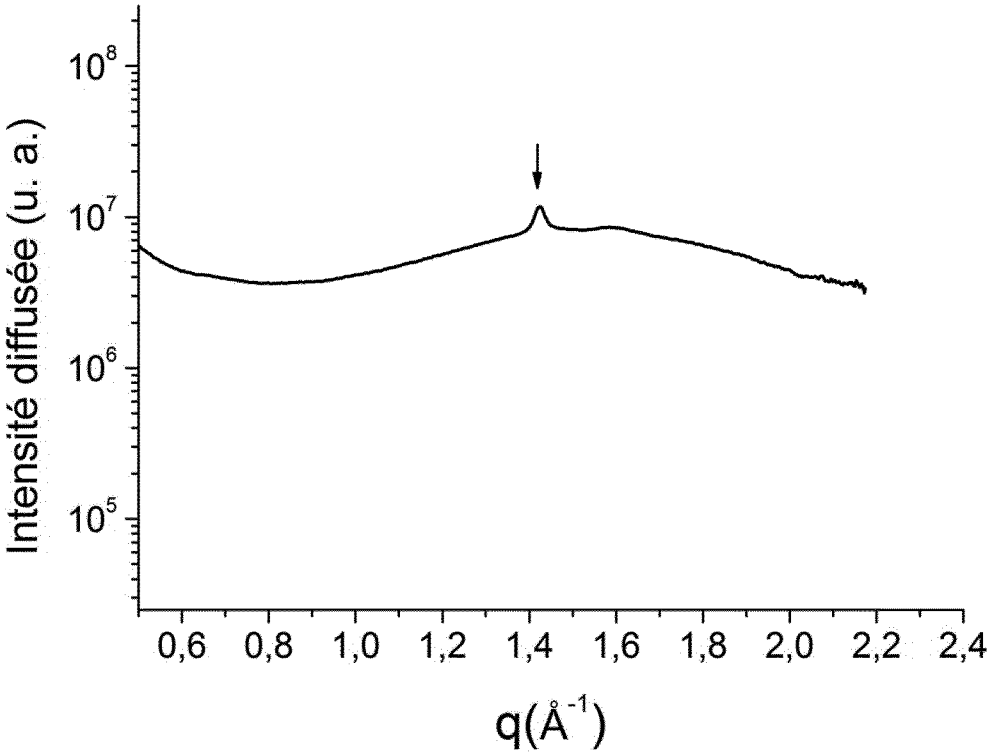


Figure 5c

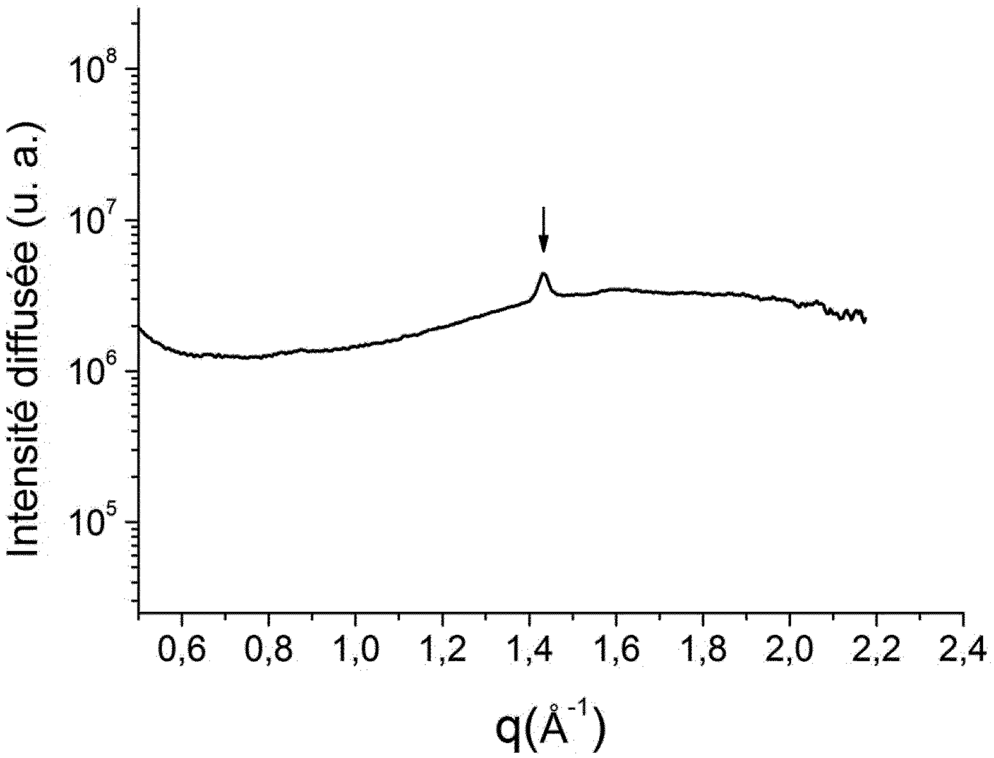


Figure 5d

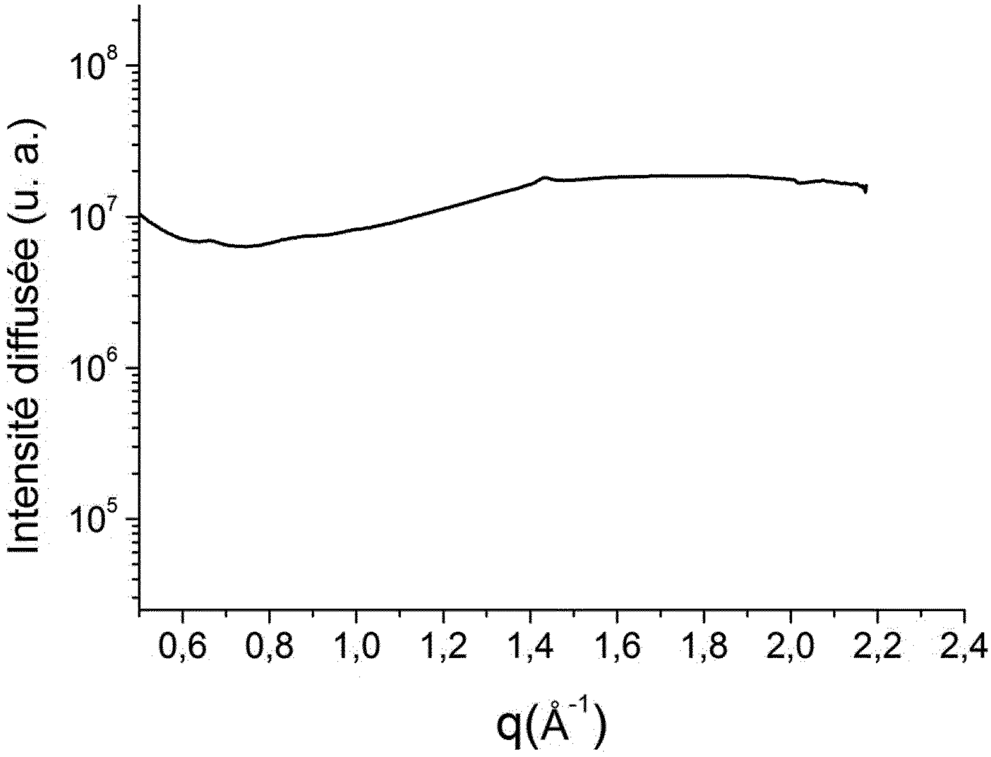


Figure 6a

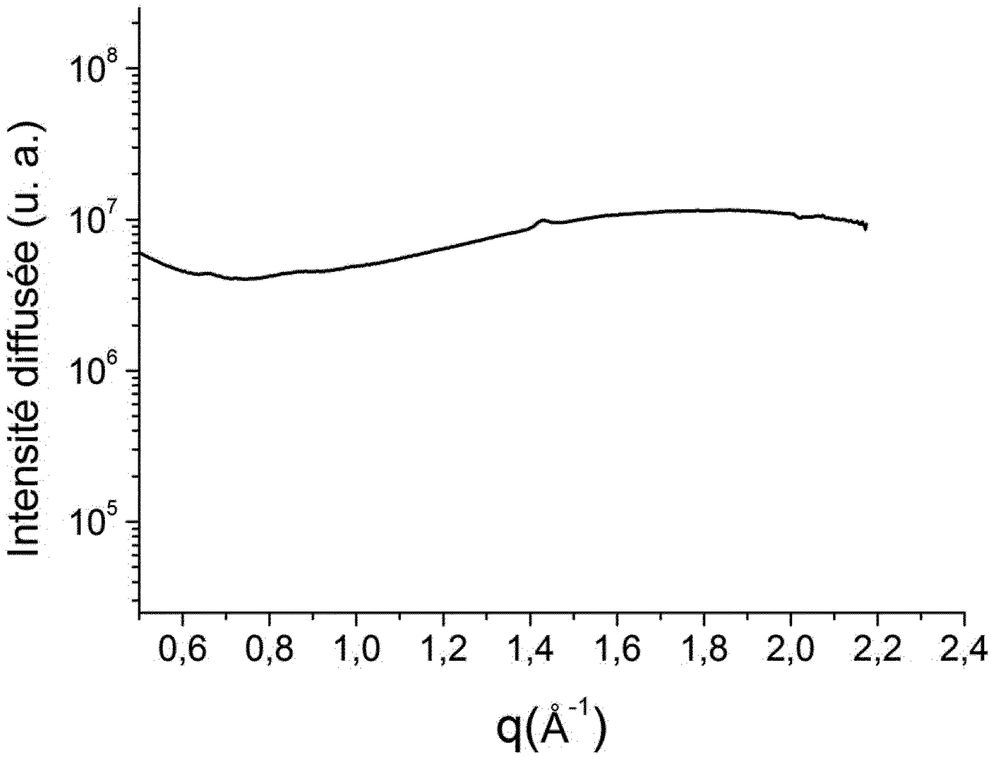


Figure 6b

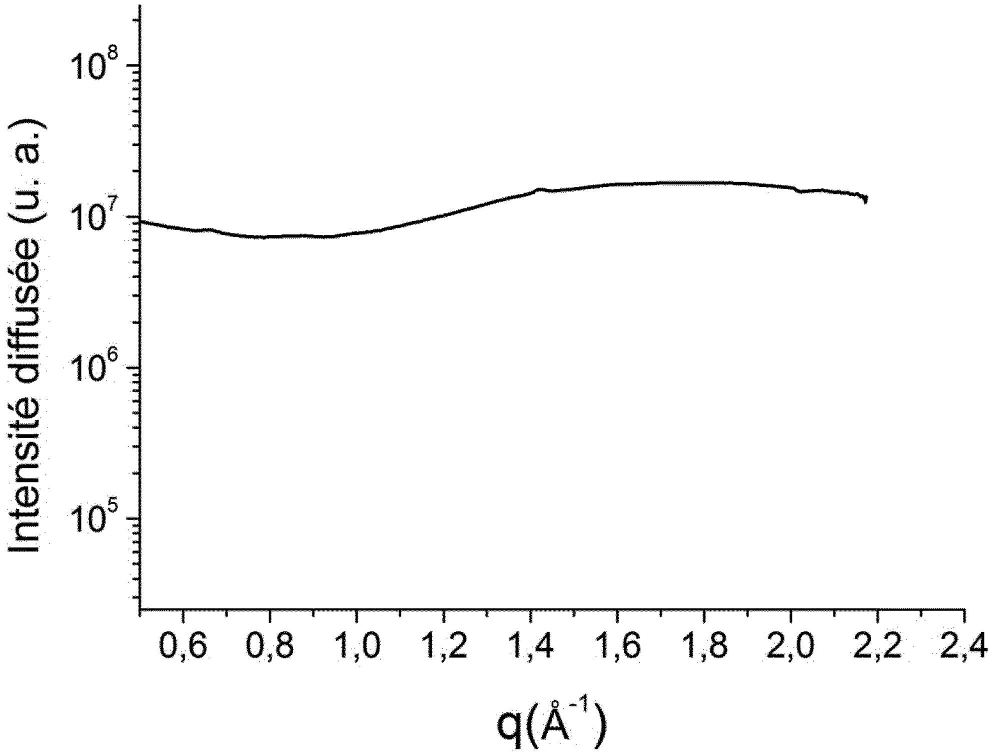


Figure 7a

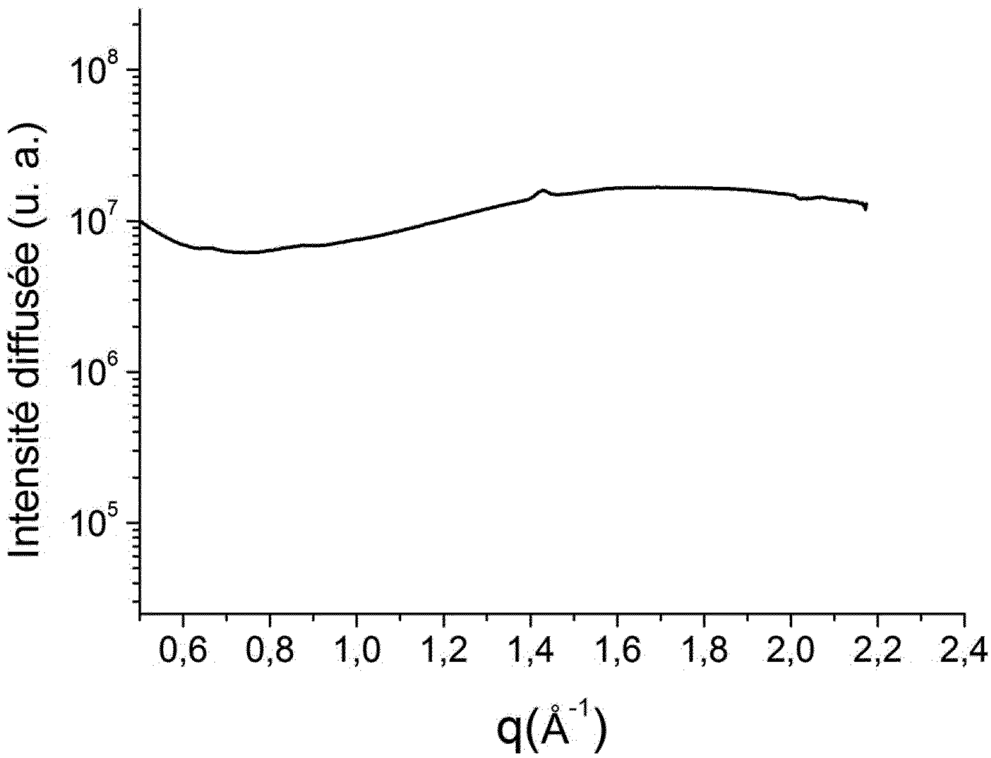


Figure 7b

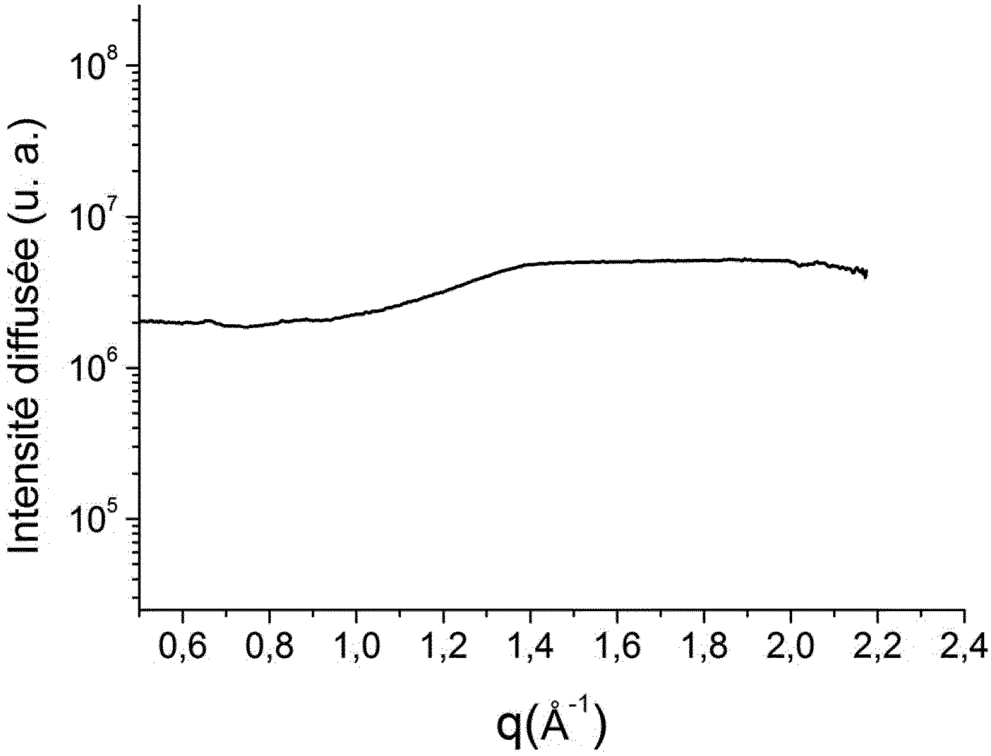


Figure 7c

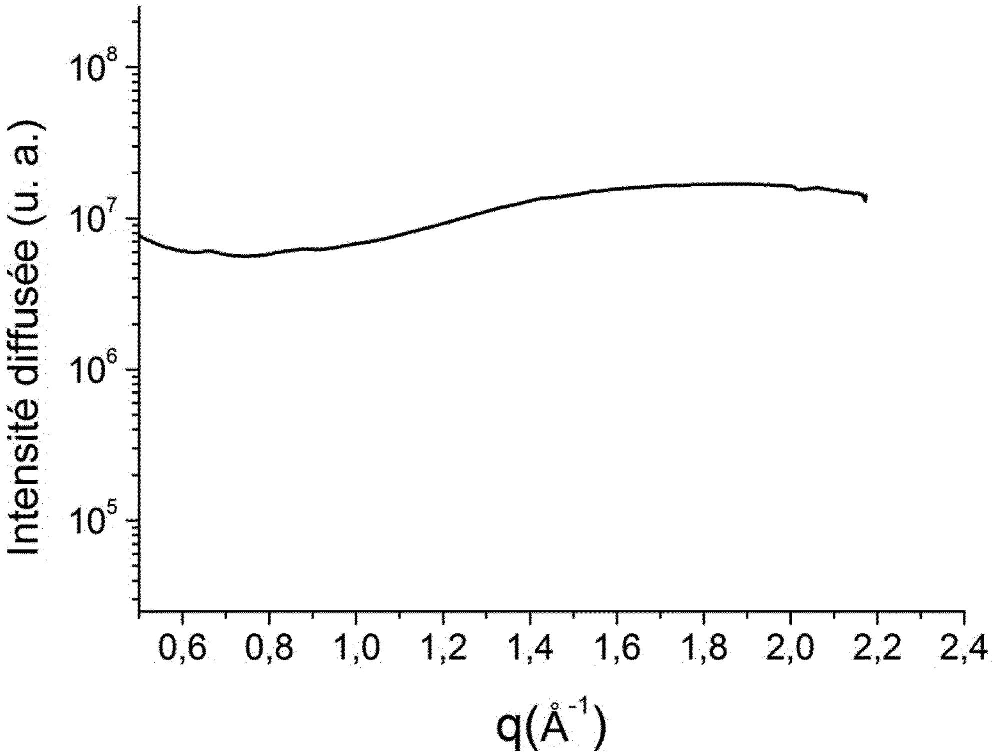


Figure 7d

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2008072230 A
- WO 2009150651 A


### Littérature non-brevet citée dans la description


- **A. MONTEMBAULT ; K. TAHIRI ; C. KORWIN-ZMIJOWSKA ; X, CHEVALIER ; M. CORVOL ; A.DOMARD.** *Biochimie,* 2006, vol. 88, 551-64
- **A. MONTEMBAULT ; C. VITON ; A. DOMARD.** Physico-chemical studies of the gelation of chitosan in a hydroalcoholic médium. *Biomaterials,* 2005, vol. 26 (8), 933-943
- **OSORIO-MADRAZO et al.** *Biomacromolecules,* 2010, vol. 11, 1376-1386